# EUROPÄISCHE PATENTSCHRIFT

(11) **EP 1 817 272 B1**
(45) Veröffentlichungstag und Bekanntmachung des Hinweises auf die Patenterteilung: **03.02.2010**
(21) Anmeldenummer: 05813485.9
(22) Anmeldetag: 01.12.2005
(51) Int. Cl.: C07C 211/54, C09B 57/00, H01L 51/00, H05B 33/00, C07F 9/50

(54) **VERBINDUNGEN F]R ORGANISCHE ELEKTRONISCHE VORRICHTUNGEN**
COMPOUNDS FOR ORGANIC ELECTRONIC DEVICES
COMPOSES DE DISPOSITIFS ELECTRONIQUES ORGANIQUES

(30) Priorität: 01.12.2004 EP 04028407
(43) Veröffentlichungstag der Anmeldung: 15.08.2007
(73) Patentinhaber: Merck Patent GmbH, 64293 Darmstadt (DE)
(72) Erfinder: VESTWEBER, Horst, 34630 Gilserberg (DE); HEUN, Susanne, 65812 Bad Soden (DE); PARHAM, Amir, 65929 Frankfurt (DE); STÖSSEL, Philipp, 60487 Frankfurt am Main (DE); HEIL, Holger, 64285 Darmstadt (DE); FORTTE, Rocco, 65933 Frankfurt (DE)
(86) Internationale Anmeldenummer: PCT/EP2005/012807
(87) Internationale Veröffentlichungsnummer: WO 2006/058737

(56) Entgegenhaltungen:
- WO-A-2004/056937
- US-A1- 2001 015 614
- PATENT ABSTRACTS OF JAPAN Bd. 2002, Nr. 04, 4. August 2002 (2002-08-04) & JP 2001 354668 A (CHISSO CORP), 25. Dezember 2001 (2001-12-25)

## Beschreibung

Die vorliegende Erfindung beschreibt neuartige Verbindungen und deren Einsatz in organischen Elektrolumineszenzvorrichtungen.

Der Einsatz halbleitender organischer Verbindungen in organischen Elektrolumineszenzvorrichtungen (OLEDs) steht gerade am Anfang der Markteinführung. Der allgemeine Aufbau solcher Vorrichtungen ist beispielsweise in US 4539507, US 5151629, EP 0676461 und WO 98/27136 beschrieben. Für einfache OLEDs enthaltende Vorrichtungen ist die Markteinführung bereits erfolgt, wie die Autoradios der Firma Pioneer, die Mobiltelefone der Firmen Pioneer und SNMD oder eine Digitalkamera der Firma Kodak mit "organischem Display" belegen. Weitere derartige Produkte stehen kurz vor der Einführung.

Allerdings zeigen diese Vorrichtungen immer noch erhebliche Probleme, die einer dringenden Verbesserung bedürfen:
1. Die operative Lebensdauer ist insbesondere bei blauer Emission immer noch gering, so dass bis dato nur einfache Anwendungen kommerziell realisiert werden konnten.
2. Die Effizienz ist in den letzten Jahren verbessert worden, ist aber gerade bei fluoreszierenden OLEDs immer noch zu niedrig und muss verbessert werden. Eine Ursache dafür könnte eine geringe Photolumineszenzquantenausbeute bei vielen der üblicherweise als blaue Emitter verwendeten Materialien sein.
3. Viele blau emittierende Verbindungen, die sowohl aromatische Amine, wie auch Doppelbindungssysteme enthalten, sind thermisch nicht stabil und zersetzen sich beim Sublimieren oder beim Aufdampfen. Dadurch ist die Verwendung dieser Systeme nicht bzw. nur unter großen Verlusten und mit hohem technischen Aufwand möglich und eine kontinuierliche Langzeitproduktion ist nicht möglich.
4. Viele blau emittierende Verbindungen, die Doppelbindungssysteme enthalten, zeigen insbesondere beim Erhitzen oder bei elektronischer Anregung Isomerisierung von der trans- zur cis-Konfiguration. Außerdem ist bekannt, dass cis-Stilben-Derivate photochemisch zu Dihydrophenanthrenen reagieren, die dann oxidativ zu Phenanthrenen weiterreagieren können. Dadurch kann es zu schlechter Reproduzierbarkeit der Vorrichtung kommen, da diese Reaktionsprodukte andere elektronische und photochemische Eigenschaften aufweisen als die Edukte.

Als nächstliegender Stand der Technik kann die Verwendung bestimmter Arylvinylamine, die an der Doppelbindung des stilbenartigen Systems nicht weiter substituiert sind, von Idemitsu genannt werden (z. B. WO 04/013073, WO 04/016575, WO 04/018587). Damit werden gute Lebensdauern bei tiefblauer Emission angegeben. Allerdings sind diese Ergebnisse stark abhängig vom verwendeten Hostmaterial, so dass die zitierten Lebensdauern nicht als Absolutwerte verglichen werden können, sondern immer nur bei Einsatz in einem optimierten Hostsystem. Weiterhin sind diese Verbindungen thermisch instabil und lassen sich nicht unzersetzt verdampfen, was daher einen hohen technischen Aufwand für die Aufdampfung erfordert und somit einen deutlichen technischen Nachteil darstellt. Einen weiteren Nachteil stellt die Emissionsfarbe dieser Verbindungen dar. Während Idemitsu tiefblaue Emission (CIE-y-Koordinaten im Bereich von 0.15-0.18) angibt, konnten diese Farbkoordinaten nicht in einfachen Vorrichtungen gemäß dem Stand der Technik reproduziert werden. Im Gegenteil erhält man hier grünblaue Emission mit CIE-y-Koordinaten im Bereich von 0.30-0.35. Es ist nicht offensichtlich, wie mit diesen Verbindungen blaue Emission erzeugt werden kann.

Wir vermuten, dass die Doppelbindung der in der Literatur beschriebenen Verbindungen zumindest für einen Teil der oben genannten Probleme verantwortlich sein könnte. So könnte die Doppelbindung beim Erhitzen (z. B. bei der Sublimation zur Reinigung der Verbindungen oder beim Aufdampfen bei der Herstellung der Vorrichtung) zur Polymerisation neigen oder könnte im angeregten Zustand bei Betrieb der Vorrichtung von der trans- zur cis-Konfiguration isomerisieren. Auch bei gleicher Substitution, bei der die Folge der Isomerisierung nicht so drastisch ist, weil das Produkt dieselbe Struktur aufweist wie das Edukt, wird dadurch die Anregungsenergie des Moleküls nicht-strahlend deaktiviert. Diese Nebenreaktionen könnten daher die Effizienz bzw. die Lebensdauer der organischen elektronischen Vorrichtung mindern. Weiterhin sind diese Nebenreaktionen möglicherweise für die geringe thermische Stabilität dieser Verbindungen verantwortlich.

Aus der JP 2001/135668 sind Triphenylamin-Derivate bekannt, welche an den para-Positionen zur Verknüpfung zum Stickstoff jeweils mit einer 2-Benzothiophen-Gruppe substituiert sind. Diese Verbindungen werden als Lochtransportmaterial in einer organischen Elektrolumineszenzvorrichtung eingesetzt.

Es besteht also weiterhin Bedarf an blau emittierenden Verbindungen, die in organischen Elektrolumineszenzvorrichtungen zu guten Effizienzen und gleichzeitig zu hohen Lebensdauern führen und die thermisch stabil und dadurch technisch unproblematisch zu verarbeiten sind. Es wurde nun überraschend gefunden, dass organische Elektrolumineszenzvorrichtungen, die bestimmte, im Folgenden aufgeführte, Verbindungen, deren Doppelbindungen durch die chemische Struktur keine cis-trans-Isomerisierung zeigen können, als blau emittierende Dotanden in einem Hostmaterial enthalten, deutliche Verbesserungen gegenüber dem Stand der Technik aufweisen. Mit diesen Materialien ist es möglich, gleichzeitig hohe Effizienzen und lange Lebensdauern zu erhalten. Auch in anderen Funktionen zeigen diese Materialien in organischen Elektrolumineszenzvorrichtungen und weiteren organischen elektronischen Vorrichtungen gute Eigenschaften. Außerdem lassen sich diese Verbindungen im Gegensatz zu Materialien gemäß dem Stand der Technik ohne merkliche Zersetzung sublimieren und aufdampfen und sind daher deutlich leichter zu handhaben als Materialien gemäß dem Stand der Technik. Diese Verbindungen und deren Verwendung in organischen elektronischen Vorrichtungen sind daher Gegenstand der vorliegenden Erfindung.

Gegenstand der Erfindung sind Verbindungen gemäß Formel (3), 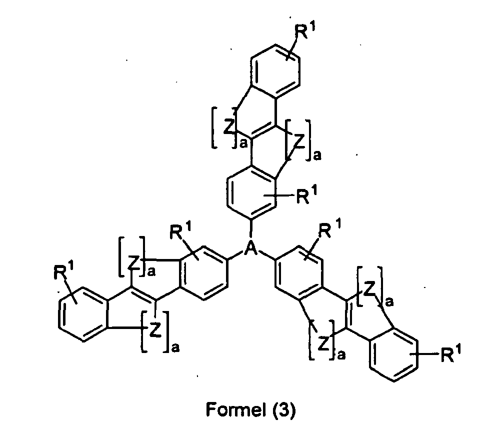 wobei für die verwendeten Symbole gilt:
- A: ist bei jedem Auftreten gleich oder verschieden N, P oder P=O;
- Z: ist bei jedem Auftreten gleich oder verschieden eine bivalente Gruppe C(R¹)₂, C=O, C[=C(R¹)₂], Si(R¹)₂, O, S=O, SO₂, NR, SR¹, PR¹, PR¹O, C(R¹)₂-C(R¹)₂, C(R¹)₂-NR¹, C(R¹)₂-C(R¹)₂-C(R¹)₂ oder C(R¹)₂-O-C(R¹)₂;
- R¹: ist bei jedem Auftreten gleich oder verschieden H, F, Cl, Br, I, CN, NO₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch -R²C=CR²-, -C≡C-, P(=O)(R²), SO, SO₂, Si(R²)₂, Ge(R²)₂, Sn(R²)₂, C=O, C=S, C=Se, C=NR², -O-, -S- oder -CONR²- ersetzt sein können und wobei ein oder mehrere H-Atome durch F, Cl, Br, I, CN oder NO₂ ersetzt sein können, oder ein aromatisches oder heteroaromatisches System mit 5 bis 40 aromatischen Ringatomen, das durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Kombination aus zwei, drei oder vier dieser Systeme; dabei können zwei oder mehrere Substituenten R¹ auch miteinander ein mono- oder polycyclisches aliphatisches Ringsystem bilden;
- R²: ist bei jedem Auftreten gleich oder verschieden H oder ein aliphatischer oder aromatischer Kohlenwasserstoffrest mit 1 bis 20 C-Atomen;
- a: ist bei jedem Auftreten gleich oder verschieden 0 oder 1, mit der Maßgabe, dass pro Doppelbindung mindestens ein Index a = 1 ist, wobei a = 0 bedeutet, dass statt Z eine Gruppe R¹ gebunden ist;
wobei die maximale Anzahl der Substituenten R¹ der Anzahl der substituierbaren H-Atome entspricht.

Auch wenn dies aus der Beschreibung hervorgeht, sei hier nochmals explizit darauf verwiesen, dass die Reste R¹ auch miteinander ein Ringsystem aufbauen können und dadurch insbesondere auch ein Spiro-System aufspannen können.

Eine Arylgruppe im Sinne dieser Erfindung enthält 6 bis 40 C-Atome; eine Heteroarylgruppe im Sinne dieser Erfindung enthält 2 bis 40 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (weniger als 10 % der von H verschiedenen Atome, bevorzugt weniger als 5 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, etc. als aromatische Ringsysteme im Sinne dieser Erfindung verstanden werden. Dabei kann ein Teil des aromatischen oder heteroaromatischen Ringsystems auch eine kondensierte Gruppe sein. Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 40 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Bevorzugt sind Verbindungen gemäß Formel (3), für die gilt:
- A: ist bei jedem Auftreten N oder P;
- Z: ist bei jedem Auftreten gleich oder verschieden C(R¹)₂, SO₂, BR¹, P(R¹)O, C(R¹)₂- C(R¹)₂, C(R¹)₂-NR¹;
- a: ist bei jedem Auftreten gleich 0 oder 1, wobei an jeder Doppelbindung ein Index a = 0 und der andere Index a = 1 ist;
- R¹ und R²: sind wie oben definiert.

Besonders bevorzugt sind Verbindungen gemäß Formel (3), für die gilt:
- A: ist bei jedem Auftreten N;
- Z: ist bei jedem Auftreten gleich oder verschieden C(R¹)₂, P(R¹)O oder C(R¹)₂-C(R¹)₂, insbesondere C(R¹)₂;
- R¹, R² und a: sind wie oben definiert.

Diese Bevorzugung gilt vor allem, wenn die Verbindung gemäß Formel (3) als blau emittierender Dotand eingesetzt wird. In anderen Funktionen in der organischen elektronischen Vorrichtung kann daher auch eine andere Auswahl der Gruppen bevorzugt sein, insbesondere der Gruppen A und Z.

Weiterhin bevorzugt sind Verbindungen gemäß Formel (3), in denen alle Einheiten Z jeweils gleich gewählt sind.
Besonders bevorzugt sind Verbindungen gemäß Formel (3), die symmetrisch aufgebaut sind und die eine dreizählige Drehachse aufweisen, insbesondere solche, in denen alle Einheiten Z und alle Substituenten R¹ und R² jeweils gleich gewählt sind.
Diese Bevorzugungen ergeben sich vor allem aus der leichteren synthetischen Zugänglichkeit der symmetrisch aufgebauten Verbindungen. Durch aufwändigere Synthesemethoden sind jedoch prinzipiell auch die unsymmetrisch substituierten Verbindungen zugänglich.

Weiterhin bevorzugt sind Verbindungen gemäß Formel (3), die keine benzylischen Protonen aufweisen. Dies gilt insbesondere für Reste an der Gruppe Z. Wenn die Gruppe Z gleich C(R¹)₂ oder C(R¹)₂C(R¹)₂ ist, ist R¹ also bevorzugt ungleich H. Diese Bevorzugung ist mit der vergleichsweise hohen Reaktivität benzylischer Protonen zu begründen, so dass die Anwesenheit benzylischer Protonen möglicherweise zu unerwünschten Nebenreaktionen bei Device-Herstellung und -Betrieb führen könnte.

Beispiele für bevorzugte Verbindungen gemäß Formel (3) sind die im Folgenden abgebildeten Strukturen (1) bis (30), die mit R¹ substituiert oder unsubstituiert sein können.

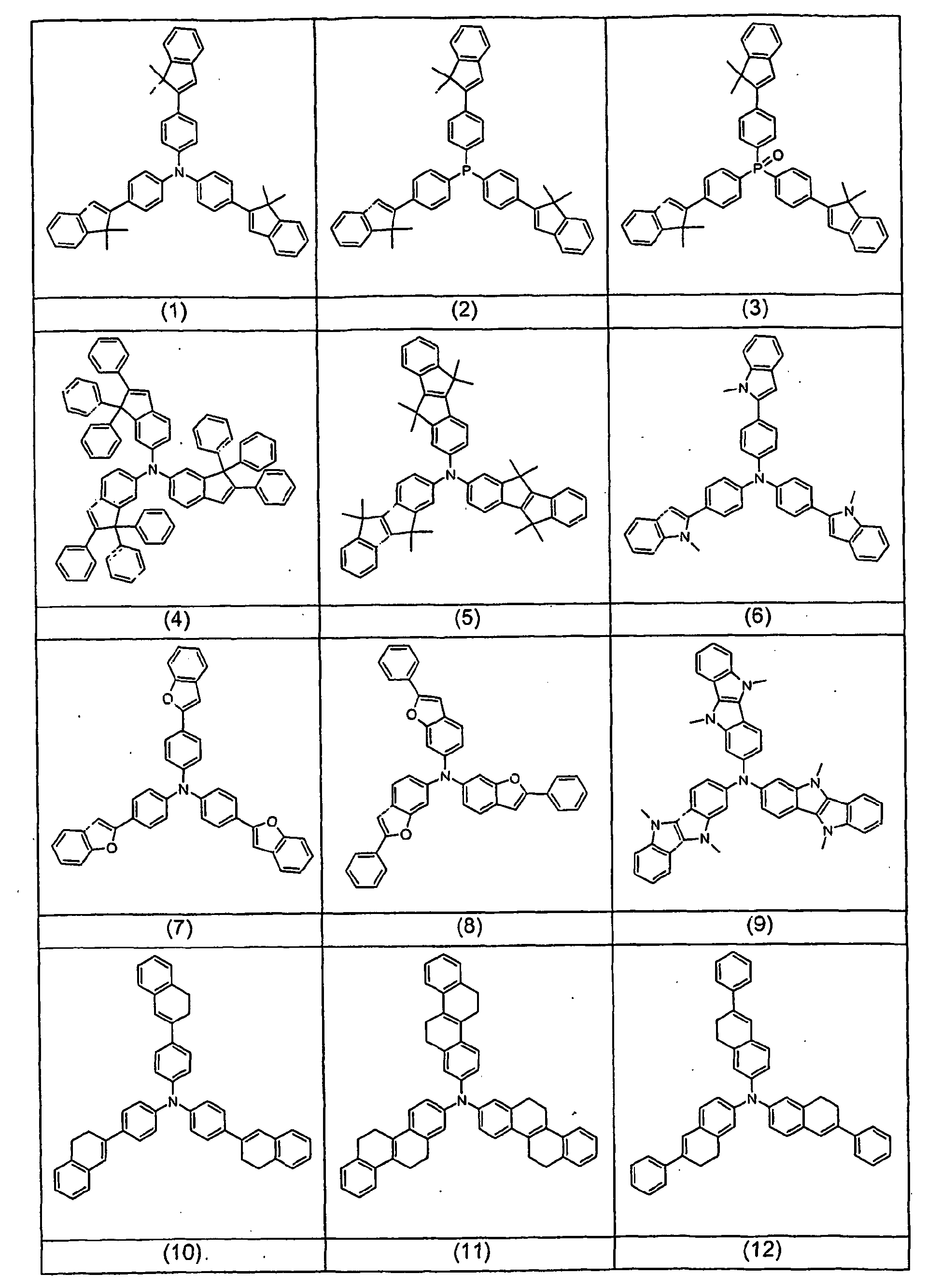

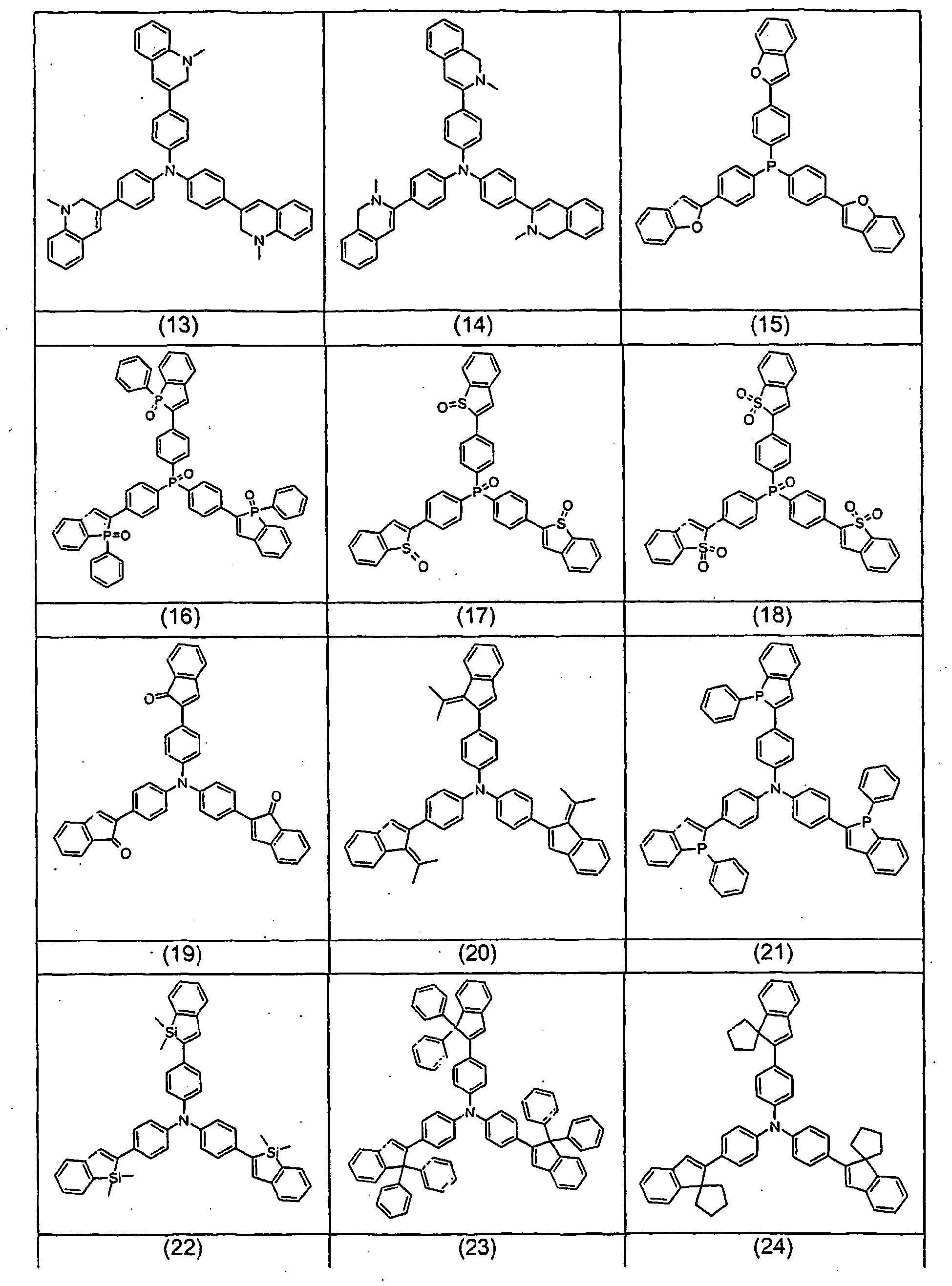

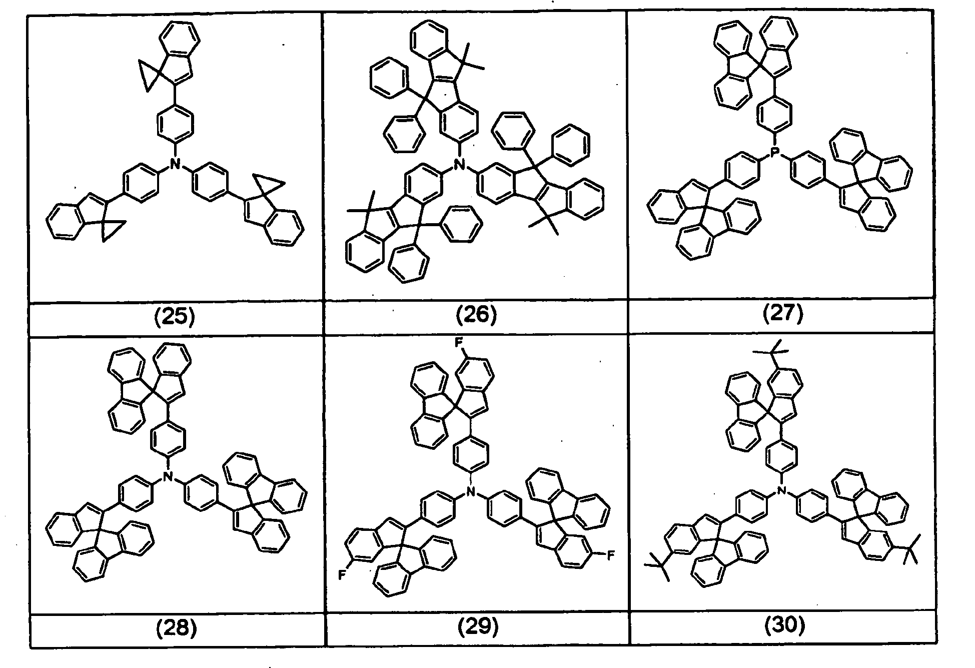

Eine ganz besonders bevorzugte Ausführungsform der Erfindung sind Verbindungen der Formeln (4), (6) und (9),

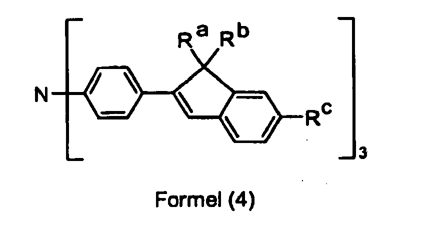

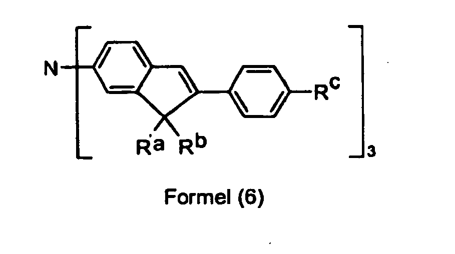

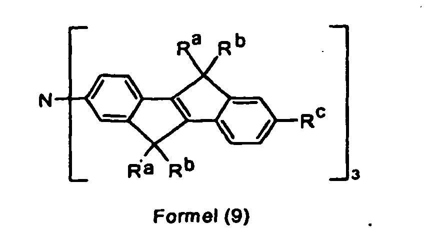

Dabei sind die Substituenten R^{a}, R^{b} und R^{c} bevorzugt aus den Substituenten gewählt, wie sie in Tabelle 1 aufgeführt sind. Dabei gelten diese bevorzugten Substituentenkombinationen für alle der Verbindungen gemäß Formel (4), (6) und (9).

**Tabelle 1: Besonders bevorzugte Substituenten an den Verbindungen gemäß Formel (4), (6) und (9)**

| Nr. | R^{a} | R^{b} | R^{c} |
|---|---|---|---|
| 1 | H | H | H |
| 2 | H | H | F |
| 3 | H | H | Methyl |
| 4 | H | H | tert-Butyl |
| 5 | H | H | Br |
| 6 | H | H | Phenyl |
| 7 | H | H | para-Tolyl |
| 8 | H | H | para-Xylyl |
| 9 | H | Methyl | H |
| 10 | H | Methyl | F |
| 11 | H | Methyl | Methyl |
| 12 | H | Methyl | tert-8utyl |
| 13 | H | Methyl | Br |
| 14 | H | Methyl | Phenyl |
| 15 | H | Methyl | para-Tolyl |
| 16 | H | Methyl | para-Xylyl |
| 17 | H | F | H |
| 18 | H | F | F |
| 19 | H | F | Methyl |
| 20 | H | F | tert-Butyl |
| 21 | H | F | Br |
| 22 | H | F | Phenyl |
| 23 | H | F | para-Tolyl |
| 24 | H | F | para-Xylyl |
| 25 | H | CF₃ | H |
| 26 | H | CF₃ | F |
| 27 | H | CF₃ | Methyl |
| 28 | H | CF₃ | tert-Butyl |
| 29 | H | CF₃ | Br |
| 30 | H | CF₃ | Phenyl |
| 31 | H | CF₃ | para-Tolyl |
| 32 | H | CF₃ | para-Xylyl |
| 33 | H | tert-Butyl | H |
| 34 | H | tert-Butyl | F |
| 35 | H | tert-Butyl | Methyl |
| 36 | H | tert-Butyl | tert-Butyl |
| 37 | H | tert-Butyl | Br |
| 38 | H | tert-Butyl | Phenyl |
| 39 | H | tert-Butyl | para-Tolyl |
| 40 | H | tert-Butyl | para-Xylyl |
| 41 | H | Phenyl | H |
| 42 | H | Phenyl | F |
| 43 | H | Phenyl | Methyl |
| 44 | H | Phenyl | tert-Butyl |
| 45 | H | Phenyl | Br |
| 46 | H | Phenyl | Phenyl |
| 47 | H | Phenyl | para-Tolyl |
| 48 | H | Phenyl | para-Xylyl |
| 49 | H | para-Tolyl | H |
| 50 | H | para-Tolyl | F |
| 51 | H | para-Tolyl | Methyl |
| 52 | H | para-Tolyl | tert-Butyl |
| 53 | H | para-Tolyl | Br |
| 54 | H | para-Tolyl | Phenyl |
| 55 | H | para-Tolyl | para-Tolyl |
| 56 | H | para-Tolyl | para-Xylyl |
| 57 | H | para-Fluor-Phenyl | H |
| 58 | H | para-Fluor-Phenyl | F |
| 59 | H | para-Fluor-Phenyl | Methyl |
| 60 | H | para-Fluor-Phenyl | tert-Butyl |
| 61 | H | para-Fluor-Phenyl | Br |
| 62 | H | para-Fluor-Phenyl | Phenyl |
| 63 | H | para-Fluor-Phenyl | para-Tolyl |
| 64 | H | para-Fluor-Phenyl | para-Xylyl |
| 65 | H | para-Xylyl | H |
| 66 | H | para-Xylyl | F |
| 67 | H | para-Xylyl | Methyl |
| 68 | H | para-Xylyl | tert-Butyl |
| 69 | H | para-Xylyl | Br |
| 70 | H | para-Xylyl | Phenyl |
| 71 | H | para-Xylyl | para-Tolyl |
| 72 | H | para-Xylyl | para-Xylyl |
| 73 | Methyl | Methyl | H |
| 74 | Methyl | Methyl | F |
| 75 | Methyl | Methyl | Methyl |
| 76 | Methyl | Methyl | tert-Butyl |
| 77 | Methyl | Methyl | Br |
| 78 | Methyl | Methyl | Phenyl |
| 79 | Methyl | Methyl | para-Tolyl |
| 80 | Methyl | Methyl | para-Xylyl |
| 81 | Methyl | F | H |
| 82 | Methyl | F | F |
| 83 | Methyl | F | Methyl |
| 84 | Methyl | F | tert-Butyl |
| 85 | Methyl | F | Br |
| 86 | Methyl | F | Phenyl |
| 87 | Methyl | F | para-Tolyl |
| 88 | Methyl | F | para-Xylyl |
| 89 | Methyl | CF₃ | H |
| 90 | Methyl | CF₃ | F |
| 91 | Methyl | CF₃ | Methyl |
| 92 | Methyl | CF₃ | tert-Butyl |
| 93 | Methyl | CF₃ | Br |
| 94 | Methyl | CF₃ | Phenyl |
| 95 | Methyl | CF₃ | para-Tolyl |
| 96 | Methyl | CF₃ | para-Xylyl |
| 97 | Methyl | tert-Butyl | H |
| 98 | Methyl | tert-Butyl | F |
| 99 | Methyl | tert-Butyl | Methyl |
| 100 | Methyl | tert-Butyl | tert-Butyl |
| 101 | Methyl | tert-Butyl | Br |
| 102 | Methyl | tert-Butyl | Phenyl |
| 103 | Methyl | tert-Butyl | para-Tolyl |
| 104 | Methyl | tert-Butyl | para-Xylyl |
| 105 | Methyl | Phenyl | H |
| 106 | Methyl | Phenyl | F |
| 107 | Methyl | Phenyl | Methyl |
| 108 | Methyl | Phenyl | tert-Butyl |
| 109 | Methyl | Phenyl | Br |
| 110 | Methyl | Phenyl | Phenyl |
| 111 | Methyl | Phenyl | para-Tolyl |
| 112 | Methyl | Phenyl | para-Xylyl |
| 113 | Methyl | para-Tolyl | H |
| 114 | Methyl | para-Tolyl | F |
| 115 | Methyl | para-Tolyl | Methyl |
| 116 | Methyl | para-Tolyl | tert-Butyl |
| 117 | Methyl | para-Tolyl | Br |
| 118 | Methyl | para-Tolyl | Phenyl |
| 119 | Methyl | para-Tolyl | para-Tolyl |
| 120 | Methyl | para-Tolyl | para-Xylyl |
| 121 | Methyl | para-Fluor-Phenyl | H |
| 122 | Methyl | para-Fluor-Phenyl | F |
| 123 | Methyl | para-Fluor-Phenyl | Methyl |
| 124 | Methyl | para-Fluor-Phenyl | tert-Butyl |
| 125 | Methyl | para-Fluor-Phenyl | Br |
| 126 | Methyl | para-Fluor-Phenyl | Phenyl |
| 127 | Methyl | para-Fluor-Phenyl | para-Tolyl |
| 128 | Methyl | para-Fluor-Phenyl | para-Xylyl |
| 129 | Methyl | para-Xylyl | H |
| 130 | Methyl | para-Xylyl | F |
| 131 | Methyl | park-xylyl | Methyl |
| 132 | Methyl | para-Xylyl | tert-Butyl |
| 133 | Methyl | para-Xylyl | Br |
| 134 | Methyl | para-Xylyl | Phenyl |
| 135 | Methyl | para-Xylyl | para-Tolyl |
| 136 | Methyl | para-Xylyl | para-Xylyl |
| 137 | F | F | H |
| 138 | F | F | F |
| 139 | F | F | Methyl |
| 140 | F | F | tert-Butyl |
| 141 | F | F | Br |
| 142 | F | F | Phenyl |
| 143 | F | F | para-Tolyl |
| 144 | F | F | para-Xylyl |
| 145 | F | CF₃ | H |
| 146 | F | CF₃ | F |
| 147 | F | CF₃ | Methyl |
| 148 | F | CF₃ | tert-Butyl |
| 149 | F | CF₃ | Br |
| 150 | F | CF₃ | Phenyl |
| 151 | F | CF₃ | para-Tolyl |
| 152 | F | CF₃ | para-Xylyl |
| 153 | F | tert-Butyl | H |
| 154 | F | tert-Butyl | F |
| 155 | F | tert-Butyl | Methyl |
| 156 | F | tert-Butyl | tert-Butyl |
| 157 | F | tert-Butyl | Br |
| 158 | F | tert-Butyl | Phenyl |
| 159 | F | tert-Butyl | para-Tolyl |
| 160 | F | tert-Butyl | para-Xylyl |
| 161 | F | Phenyl | H |
| 162 | F | Phenyl | F |
| 163 | F | Phenyl | Methyl |
| 164 | F | Phenyl | tert-Butyl |
| 165 | F | Phenyl | Br |
| 166 | F | Phenyl | Phenyl |
| 167 | F | Phenyl | para-Tolyl |
| 168 | F | Phenyl | para-Xylyl |
| 169 | F | para-Tolyl | H |
| 170 | F | para-Tolyl | F |
| 171 | F | para-Tolyl | Methyl |
| 172 | F | para-Tolyl | tert-Butyl |
| 173 | F | para-Tolyl | Br |
| 174 | F | para-Tolyl | Phenyl |
| 175 | F | para-Tolyl | para-Tolyl |
| 176 | F | para-Tolyl | para-Xylyl |
| 177 | F | para-Fluor-Phenyl | H |
| 178 | F | para-Fluor-Phenyl | F |
| 179 | F | para-Fluor-Phenyl | Methyl |
| 180 | F | para-Fluor-Phenyl | tert-Butyl |
| 181 | F | para-Fluor-Phenyl | Br |
| 182 | F | para-Fluor-Phenyl | Phenyl |
| 183 | F | para-Fluor-Phenyl | para-Tolyl |
| 184 | F | para-Fluor-Phenyl | para-Xylyl |
| 185 | F | para-Xylyl | H |
| 186 | F | para-Xylyl | F |
| 187 | F | para-Xylyl | Methyl |
| 188 | F | para-Xylyl | tert-Butyl |
| 189 | F | para-Xylyl | Br |
| 190 | F | para-Xylyl | Phenyl |
| 191 | F | para-Xylyl | para-Tolyl |
| 192 | F | para-Xylyl | para-Xylyl |
| 193 | CF₃ | CF₃ | H |
| 194 | CF₃ | CF₃ | F |
| 195 | CF₃ | CF₃ | Methyl |
| 196 | CF₃ | CF₃ | tert-Butyl |
| 197 | CF₃ | CF₃ | Br |
| 198 | CF₃ | CF₃ | Phenyl |
| 199 | CF₃ | CF₃ | para-Tolyl |
| 200 | CF₃ | CF₃ | para-Xylyl |
| 201 | CF₃ | tert-Butyl | H |
| 202 | CF₃ | tert-Butyl | F |
| 203 | CF₃ | tert-Butyl | Methyl |
| 204 | CF₃ | tert-Butyl | tert-Butyl |
| 205 | CF₃ | tert-Butyl | Br |
| 206 | CF₃ | tert-Butyl | Phenyl |
| 207 | CF₃ | tert-Butyl | para-Tolyl |
| 208 | CF₃ | tert-Butyl | para-Xylyl |
| 209 | CF₃ | Phenyl | H |
| 210 | CF₃ | Phenyl | F |
| 211 | CF₃ | Phenyl | Methyl |
| 212 | CF₃ | Phenyl | tert-Butyl |
| 213 | CF₃ | Phenyl | Br |
| 214 | CF₃ | Phenyl | Phenyl |
| 215 | CF₃ | Phenyl | para-Tolyl |
| 216 | CF₃ | Phenyl | para-Xylyl |
| 217 | CF₃ | para-Tolyl | H |
| 218 | CF₃ | para-Tolyl | F |
| 219 | CF₃ | para-Tolyl | Methyl |
| 220 | CF₃ | para-Tolyl | tert-Butyl |
| 221 | CF₃ | para-Tolyl | Br |
| 222 | CF₃ | para-Tolyl | Phenyl |
| 223 | CF₃ | para-Tolyl | para-Tolyl |
| 224 | CF₃ | para-Tolyl | para-Xylyl |
| 225 | CF₃ | para-Fluor-Phenyl | H |
| 226 | CF₃ | para-Fluor-Phenyl | F |
| 227 | CF₃ | para-Fluor-Phenyl | Methyl |
| 228 | CF₃ | para-Fluor-Phenyl | tert-Butyl |
| 229 | CF₃ | para-Fluor-Phenyl | Br |
| 230 | CF₃ | para-Fluor-Phenyl | Phenyl |
| 231 | CF₃ | para-Fluor-Phenyl | para-Tolyl |
| 232 | CF₃ | para-Fluor-Phenyl | para-Xylyl |
| 233 | CF₃ | para-Xylyl | H |
| 234 | CF₃ | para-Xylyl | F |
| 235 | CF₃ | para-Xylyl | Methyl |
| 236 | CF₃ | para-Xylyl | tert-Butyl |
| 237 | CF₃ | para-Xylyl | Br |
| 238 | CF₃ | para-Xylyl | Phenyl |
| 239 | CF₃ | para-Xylyl | para-Tolyl |
| 240 | CF₃ | para-Xylyl | para-Xylyl |
| 241 | tert-Butyl | tert-Butyl | H |
| 242 | tert-Butyl | tert-Butyl | F |
| 243 | tert-Butyl | tert-Butyl | Methyl |
| 244 | tert-Butyl | tert-Butyl | tert-Butyl |
| 245 | tert-Butyl | tert-Butyl | Br |
| 246 | tert-Butyl | tert-Butyl | Phenyl |
| 247 | tert-Butyl | tert-Butyl | para-Tolyl |
| 248 | tert-Butyl | tert-Butyl | para-Xylyl |
| 249 | tert-Butyl | Phenyl | H |
| 250 | tert-Butyl | Phenyl | F |
| 251 | tert-Butyl | Phenyl | Methyl |
| 252 | tert-Butyl | Phenyl | tert-Butyl |
| 253 | tert-Butyl | Phenyl | Br |
| 254 | tert-Butyl | Phenyl | Phenyl |
| 255 | tert-Butyl | Phenyl | para-Tolyl |
| 256 | tert-Butyl | Phenyl | para-Xylyl |
| 257 | tert-Butyl | para-Tolyl | H |
| 258 | tert-Butyl | para-Tolyl | F |
| 259 | tert-Butyl | para-Tolyl | Methyl |
| 260 | tert-Butyl | para-Tolyl | tert-Butyl |
| 261 | tert-Butyl | para-Tolyl | Br |
| 262 | tert-Butyl | para-Tolyl | Phenyl |
| 263 | tert-Butyl | para-Tolyl | para-Tolyl |
| 264 | tert-Butyl | para-Tolyl | para-Xylyl |
| 265 | tert-Butyl | para-Fluor-Phenyl | H |
| 266 | tert-Butyl | para-Fluor-Phenyl | F |
| 267 | tert-Butyl | para-Fluor-Phenyl | Methyl |
| 268 | tert-Butyl | para-Fluor-Phenyl | tert-Butyl |
| 269 | tert-Butyl | para-Fluor-Phenyl | Br |
| 270 | tert-Butyl | para-Fluor-Phenyl | Phenyl |
| 271 | tert-Butyl | para-Fluor-Phenyl | para-Tolyl |
| 272 | tert-Butyl | para-Fluor-Phenyl | para-Xylyl |
| 273 | tert-Butyl | para-Xylyl | H |
| 274 | tert-Butyl | para-Xylyl | F |
| 275 | tert-Butyl | para-Xylyl | Methyl |
| 276 | tert-Butyl | para-Xylyl | tert-Butyl |
| 277 | tert-Butyl | para-Xylyl | Br |
| 278 | tert-Butyl | para-Xylyl | Phenyl |
| 279 | tert-Butyl | para-Xylyl | para-Tolyl |
| 280 | tert-Butyl | para-Xylyl | para-Xylyl |
| 281 | Phenyl | Phenyl | H |
| 282 | Phenyl | Phenyl | F |
| 283 | Phenyl | Phenyl | Methyl |
| 284 | Phenyl | Phenyl | tert-Butyl |
| 285 | Phenyl | Phenyl | Br |
| 286 | Phenyl | Phenyl | Phenyl |
| 287 | Phenyl | Phenyl | para-Tolyl |
| 288 | Phenyl | Phenyl | para-Xylyl |
| 289 | Phenyl | para-Tolyl | H |
| 290 | Phenyl | para-Tolyl | F |
| 291 | Phenyl | para-Tolyl | Methyl |
| 292 | Phenyl | para-Tolyl | tert-Butyl |
| 293 | Phenyl | para-Tolyl | Br |
| 294 | Phenyl | para-Tolyl | Phenyl |
| 295 | Phenyl | para-Tolyl | para-Tolyl |
| 296 | Phenyl | para-Tolyl | para-Xylyl |
| 297 | Phenyl | para-Fluor-Phenyl | H |
| 298 | Phenyl | para-Fluor-Phenyl | F |
| 299 | Phenyl | para-Fluor-Phenyl | Methyl |
| 300 | Phenyl | para-Fluor-Phenyl | tert-Butyl |
| 301 | Phenyl | para-Fluor-Phenyl | Br |
| 302 | Phenyl | para-Fluor-Phenyl | Phenyl |
| 303 | Phenyl | para-Fluor-Phenyl | para-Tolyl |
| 304 | Phenyl | para-Fluor-Phenyl | para-Xylyl |
| 305 | Phenyl | para-Xylyl | H |
| 306 | Phenyl | para-Xylyl | F |
| 307 | Phenyl | para-Xylyl | Methyl |
| 308 | Phenyl | para-Xylyl | tert-Butyl |
| 309 | Phenyl | para-Xylyl | Br |
| 310 | Phenyl | para-Xylyi | Phenyl |
| 311 | Phenyl | para-Xylyl | para-Tolyl |
| 312 | Phenyl | para-Xylyl | para-Xylyl |
| 313 | para-Tolyl | para-Tolyl | H |
| 314 | para-Tolyl | para-Tolyl | F |
| 315 | para-Tolyl | para-Tolyl | Methyl |
| 316 | para-Tolyl | para-Tolyl | tert-Butyl |
| 317 | para-Tolyl | para-Tolyl | Br |
| 318 | para-Tolyl | para-Tolyl | Phenyl |
| 319 | para-Tolyl | para-Tolyl | para-Tolyl |
| 320 | para-Tolyl | para-Tolyl | para-Xylyl |
| 321 | para-Tolyl | para-Fluor-Phenyl | H |
| 322 | para-Tolyl | para-Fluor-Phenyl | F |
| 323 | para-Tolyl | para-Fluor-Phenyl | Methyl |
| 324 | para-Tolyl | para-Fluor-Phenyl | tert-Butyl |
| 325 | para-Tolyl | para-Fluor-Phenyl | Br |
| 326 | para-Tolyl | para-Fluor-Phenyl | Phenyl |
| 327 | para-Tolyl | para-Fluor-Phenyl | para-Tolyl |
| 328 | para-Tolyl | para-Fluor-Phenyl | para-Xylyl |
| 329 | para-Tolyl | para-Xylyl | H |
| 330 | para-Tolyl | para-Xylyl | F |
| 331 | para-Tolyl | para-Xylyl | Methyl |
| 332 | para-Tolyl | para-Xylyl | tert-Butyl |
| 333 | para-Tolyl | para-Xylyl | Br |
| 334 | para-Tolyl | para-Xylyl | Phenyl |
| 335 | para-Tolyl | para-Xylyl | para-Tolyl |
| 336 | para-Tolyl | para-Xylyl | para-Xylyl |
| 337 | para-Fluor-Phenyl | para-Fluor-Phenyl | H |
| 338 | para-Fluor-Phenyl | para-Fluor-Phenyl | F |
| 339 | para-Fluor-Phenyl | para-Fluor-Phenyl | Methyl |
| 340 | para-Fluor-Phenyl | para-Fluor-Phenyl | tert-Butyl |
| 341 | para-Fluor-Phenyl | para-Fluor-Phenyl | Br |
| 342 | para-Fluor-Phenyl | para-Fluor-Phenyl | Phenyl |
| 343 | para-Fluor-Phenyl | para-Fluor-Phenyl | para-Tolyl |
| 344 | para-Fluor-Phenyl | para-Fluor-Phenyl | para-Xylyl |
| 345 | para-Fluor-Phenyl | para-Xylyl | H |
| 346 | para-Fluor-Phenyl | para-Xylyl | F |
| 347 | para-Fluor-Phenyl | para-Xylyl | Methyl |
| 348 | para-Fluor-Phenyl | para-Xylyl | tert-Butyl |
| 349 | para-Fluor-Phenyl | para-Xylyl | Br |
| 350 | para-Fluor-Phenyl | para-Xylyl | Phenyl |
| 351 | para-Fluor-Phenyl | para-Xylyl | para-Tolyl |
| 352 | para-Fluor-Phenyl | para-Xylyl | para-Xylyl |
| 353 | para-Xylyl | para-Xylyl | H |
| 354 | para-Xylyl | para-Xylyl | F |
| 355 | para-Xylyl | para-Xylyl | Methyl |
| 356 | para-Xylyl | para-Xylyl | tert-Butyl |
| 357 | para-Xylyl | para-Xylyl | Br |
| 358 | para-Xylyl | para-Xylyl | Phenyl |
| 359 | para-Xylyl | para-Xylyl | para-Tolyl |
| 360 | para-Xylyl | para-Xylyl | para-Xylyl |
| 361 | 2,2'-Biphenylyl | | H |
| 362 | 2,2'-Biphenylyl | | F |
| 363 | 2,2'-Biphenylyl | | Methyl |
| 364 | 2;2'-Biphenylyl | | tert-Butyl |
| 365 | 2,2'-Biphenylyl | | Br |
| 366 | 2,2'-Biphenylyl | | Phenyl |
| 367 | 2,2'-Biphenylyl | | para-Tolyl |
| 368 | 2,2'-Biphenylyl | | para-Xylyl |

Ein weiterer Gegenstand der vorliegenden Erfindung ist die Verwendung von Verbindungen gemäß Formel (3) in organischen elektronischen Vorrichtungen, insbesondere in organischen Elektrolumineszenzvorrichtungen.

Gegenstand der Erfindung sind weiterhin organische Elektrolumineszenzvorrichtungen, enthaltend Kathode, Anode und mindestens eine emittierende Schicht, dadurch gekennzeichnet, dass mindestens eine organische Schicht mindestens eine Verbindung gemäß Formel (3) enthält.

Außer der emittierenden Schicht kann die organische Elektrolumineszenzvorrichtung noch weitere Schichten enthalten. Diese können beispielsweise sein: Lochinjektionsschicht, Lochtransportschicht, Lochblockierschicht, Elektronentransportschicht und/oder Elektroneninjektionsschicht. Es sei aber an dieser Stelle darauf hingewiesen, dass nicht notwendigerweise jede dieser Schichten vorhanden sein muss. So werden insbesondere bei Verwendung von Verbindungen gemäß Formel (3) als Dotand mit elektronenleitenden Hostmaterialien weiterhin sehr gute Ergebnisse erhalten, wenn die organische Elektrolumineszenzvorrichtung keine separate Elektronentransportschicht enthält und die emittierende Schicht direkt an die Elektroneninjektionsschicht oder an die Kathode grenzt. Ebenfalls kann es bevorzugt sein, wenn die organische Elektrolumineszenzvorrichtung keine separate Lochtransportschicht enthält und die emittierende Schicht direkt an die Lochinjektionsschicht oder an die Anode grenzt. Weiterhin kann es bevorzugt sein, wenn die Verbindung gemäß Formel (3) nicht nur als Dotand in der emittierenden Schicht, sondern auch zusätzlich als lochleitende Verbindung (als Reinsubstanz oder als Mischung) in der Lochtransportschicht verwendet wird.

Die Verbindung gemäß Formel (3) kann unterschiedliche Funktionen in der organischen Elektrolumineszenzvorrichtung wahrnehmen. Diese hängen von der genauen Struktur dieser Verbindung ab. Insbesondere die Wahl der Gruppen A und Z bestimmen die besonders geeignete Funktion dieser Verbindungen. So können diese Verbindungen insbesondere als Emitter, als Lochtransportmaterial, als Elektronentransportmaterial oder in elektrophosphoreszierenden Vorrichtungen auch als Matrixmaterial bzw. als Lochblockiermaterial eingesetzt werden.

Als Emitter eignen sich insbesondere Verbindungen, in denen für die Symbole A und Z gilt:
- A: ist bei jedem Auftreten N;
- Z: ist bei jedem Auftreten gleich oder verschieden C(R¹)₂, C(R¹)₂-C(R¹)₂ oder C(R¹)₂NR¹.

Als Lochtransportmaterial eignen sich insbesondere Verbindungen, in denen für die Symbole A und Z gilt:
- A: ist bei jedem Auftreten N oder P;
- Z: ist bei jedem Aufteten gleich oder verschieden C(R¹)₂, C(R¹)₂-C(R¹)₂, C(R¹)₂-NR¹, O, NR¹ oder PR¹, insbesondere O, NR¹ oder PR¹.

Als Elektronentransportmaterial für fluoreszierende oder phosphoreszierende Vorrichtungen eignen sich insbesondere Verbindungen, in denen für die Symbole A und Z gilt:
- A: ist bei jedem Auftreten P=O;
- Z: ist bei jedem Auftreten gleich oder verschieden C(R¹)₂, C(R¹)₂-C(R¹)₂, C(R¹)₂-NR¹, C=O, S=O, SO₂, BR' oder PR¹O, insbesondere C=O, S=O, SO₂, BR¹ oder PR¹O.

Als Matrixmaterial und als Lochblockiermaterial für elektrophosphoreszierende Vorrichtungen eignen sich insbesondere Verbindungen, in denen für die Symbole A und Z gilt:
- A: ist bei jedem Auftreten P=O;
- Z: ist bei jedem Auftreten gleich oder verschieden C(R¹)₂, C(R¹)₂-C(R¹)₂, C(R¹)₂-NR¹, C=O, S=O, SO₂ oder PR¹O, insbesondere C=O, S=O, SO₂ oder PR¹O.

Wird die Verbindung gemäß Formel (3) als Emitter eingesetzt, so wird sie bevorzugt zusammen mit einem Host-Material verwendet. Der Anteil der Verbindung gemäß Formel (3) in der Mischung beträgt dann zwischen 0.1 und 99 Gew.%, bevorzugt zwischen 0.5 und 50 Gew.%, besonders bevorzugt zwischen 1 und 20 Gew.%, insbesondere zwischen 1 und 10 Gew.%. Entsprechend beträgt der Anteil des Hostmaterials in der Mischung zwischen 1 und 99.9 Gew.%, bevorzugt zwischen 50 und 99.5 Gew.%, besonders bevorzugt zwischen 80 und 99 Gew.%, insbesondere zwischen 90 und 99 Gew.%.

Weiterhin bevorzugt sind organische Elektrolumineszenzvorrichtungen, dadurch gekennzeichnet, dass mehrere emittierende Verbindungen in derselben Schicht oder in unterschiedlichen Schichten verwendet werden, wovon mindestens eine dieser Verbindungen eine Struktur gemäß Formel (3) aufweist. Besonders bevorzugt weisen diese Verbindungen insgesamt mehrere Emissionsmaxima zwischen 380 nm und 750 nm auf, so dass insgesamt weiße Emission resultiert, d. h. außer der Verbindung gemäß Formel (1) wird noch mindestens eine weitere emittierende Verbindung verwendet, die fluoresziert oder phosphoresziert und die gelbes, orange oder rotes Licht emittiert.

Bevorzugte Hostmaterialien sind organische Verbindungen, deren Emission kürzerwellig ist als die der Verbindung gemäß Formel (3) oder die überhaupt nicht im sichtbaren Bereich emittieren. Als Hostmaterialien kommen verschiedene Stoffklassen in Frage. Bevorzugte Hostmaterialien sind ausgewählt aus den Klassen der Oligo-arylene (z. B. 2,2',7,7'-tetraphenyl-spirobifluoren gemäß EP 676461 oder Dinaphthylanthracen), der Atropisomere (z. B. gemäß der nicht offen gelegten Anmeldung EP 04026402.0), der Oligo-arylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911) oder der elektronenleitenden Verbindungen, insbesondere Ketone, Phosphinoxide und Sulfoxide (z. B. gemäß der nicht offen gelegten Patentanmeldung DE 102004008304.5). Besonders bevorzugte Hostmaterialien sind ausgewählt aus den Klassen der Oligoarylene, enthaltend Naphthalin, Anthracen und/oder Pyren, der Oligo-arylenvinylene, der Ketone, der Phosphinoxide und der Sulfoxide.

Wird die Verbindung gemäß Formel (3) als Lochtransportmaterial, Elektronentransportmaterial oder als Lochblockiermaterial eingesetzt, ist es bevorzugt, wenn diese Verbindung als Reinsubstanz eingesetzt wird. Insbesondere als Lochtransportmaterial und als Elektronentransportmaterial eignen sich diese Verbindungen auch für die Verwendung in weiteren organischen elektronischen Vorrichtungen, wie beispielsweise in organischen Transistoren.

Wird die Verbindung gemäß Formel (3) als Matrixmaterial für elektrophosphoreszierende Vorrichtungen eingesetzt, so beträgt ihr Anteil in der Mischung zwischen 1 und 99.9 Gew.%, bevorzugt zwischen 30 und 99.5 Gew.%, besonders bevorzugt zwischen 50 und 99 Gew.%, insbesondere zwischen 80 und 99 Gew.%. Entsprechend beträgt der Anteil des Emitters, der aus dem Triplett-Zustand Licht emittiert und daher Elektrophosphoreszenz zeigt, in der Mischung zwischen 0.1 und 99 Gew.%, bevorzugt zwischen 0.5 und 70 Gew.%, besonders bevorzugt zwischen 1 und 50 Gew,%, insbesondere zwischen 1 und 20 Gew.%.

Die Mischungsverhältnisse lassen sich durch Mischen in Lösemitteln (bzw. Lösemittelgemischen) oder durch Co-Verdampfen im Unterdruck, Trägergasstrom oder Vakuum einstellen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden.

Die oben beschriebenen Verbindungen weisen in organischen elektronischen Vorrichtungen folgende überraschende Vorteile gegenüber dem Stand der Technik auf:
1. Die Effizienz entsprechender Vorrichtungen ist höher im Vergleich zu Systemen gemäß dem Stand der Technik. Wir vermuten, dass dies durch die unterdrückte cis-trans-Isomerisierung um die Doppelbindung zustande kommt.
2. Die Stabilität entsprechender Vorrichtungen ist besser im Vergleich zu Systemen gemäß dem Stand der Technik, was sich vor allem in einer höheren Lebensdauer zeigt. Diese höhere Stabilität kommt möglicherweise auch durch die gehinderte cis-trans-Isomerisierung zustande, da bekannt ist, dass cis-Stilbensysteme, die entstehen können, wenn die Isomerisierung möglich ist, sich photochemisch weiter umsetzen zu Dihydrophenanthrensystemen und dann durch oxidative Folgereaktion zu Phenanthrensystemen. Diese Nebenreaktion ist in den erfindungsgemäßen Systemen nicht möglich.
3. Die Verbindungen lassen sich ohne deutliche Zersetzung sublimieren und aufdampfen, sind dadurch leichter zu reinigen und zu verarbeiten und deshalb besser für die Verwendung in OLEDs geeignet als Materialien gemäß dem Stand der Technik, insbesondere als Materialien, die Stilbeneinheiten enthalten, die an der Doppelbindung nicht substituiert sind.
4. Da die erfindungsgemäßen Verbindungen bei der Herstellung bzw. Verarbeitung bzw. im Betrieb der elektronischen Vorrichtung keine Isomerisierung erleiden, erhöht sich die Reproduzierbarkeit der Vorrichtung.

Im vorliegenden Anmeldetext und auch in den im Weiteren folgenden Beispielen wird auf die Verwendung erfindungsgemäßer Verbindungen in Bezug auf OLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch für weitere Verwendungen in anderen elektronischen Vorrichtungen zu benutzen, z. B. für organische Feld-Effekt-Transistoren (O-FETs), organische Dünnfilmtransistoren (O-TFTs), organische integrierte Schaltungen (O-ICs), organische Solarzellen (O-SCs), organische lichtemittierende Transistoren (O-LETs), organische Feld-Quench-Devices (O-FQDs), lichtemittierende elektrochemische Zellen (LECs) oder auch organische Laserdioden (O-Laser), um nur einige Anwendungen zu nennen.
Die Verwendung der erfindungsgemäßen Verbindungen in den entsprechenden Vorrichtungen ebenso wie diese Vorrichtungen selbst sind ebenfalls ein Gegenstand der vorliegenden Erfindung.
Die Erfindung wird durch die nachfolgenden Beispiele näher erläutert, ohne sie dadurch einschränken zu wollen.

### Beispiele:

Die nachfolgenden Synthesen wurden, sofern nicht anders angegeben, unter einer Schutzgasatmosphäre durchgeführt. Die Edukte wurden von ALDRICH bezogen. Die Synthese von Tris(p-bromphenyl)amin erfolgte gemäß J. Am. Chem. Soc. 1987, 109, 4960-4968. Die Synthese von 2-Indenylboronsäure erfolgte gemäß J. Org. Chem. 2002, 67, 169-176, die von 2-Brombenzyl-diethylphosphonat erfolgte gemäß Z. Anorg. Allg. Chem. 1994, 620(12), 2041-7.

### Beispiel 1: Synthese von 4,4',4" Tris(1H-inden-2-yl)triphenylamin (Amin1)

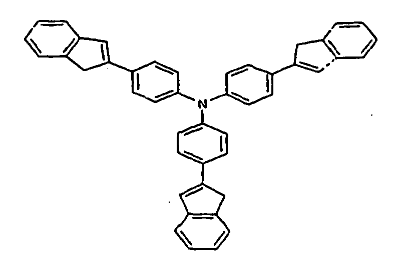

Zur einer mit Stickstoff gesättigten Mischung aus 15.0 g (31 mmol) Tris(p-bromphenyl)amin, 19.8 g (124 mmol) 2-Indenylboronsäure, 40.0 g (188 mmol) K₃PO₄, 1 L Dioxan und 1 L Wasser werden 800 mg (0.72 mmol) Pd(PPh₃)₄ gegeben. Die Suspension wird 7 h auf 80°C erhitzt. Danach werden 0.09 g NaCN zugegeben und die wässrige Phase abgetrennt. Die organische Phase wird zweimal mit H₂O gewaschen und anschließend über Na₂SO₄ getrocknet. Nach dem Entfernen des Lösemittels und mehrmaliger Umkristallisation aus Toluol erhält man gelbe Nadeln, die nach HPLC eine Reinheit von ca. 99.9 % aufweisen. Die Ausbeute beträgt 16 g (88 %).
¹H-NMR (CDCl₃, 500 MHz): δ [ppm] = 3.78 (s, 6H), 7.13-7.19 (m, 12H), 7.23-7.28 (m, 3H), 7.38 (d, J = 7.4 Hz, 3H), 7.46 (d, J = 7.4 Hz, 3H), 7.56 (d, J = 8.7 Hz, 6H).

### Beispiel 2: Synthese von 4,4',4"-Tris(1,1,3-trimethyl-1H-inden-2-yl)triphenylamin (Amin 2)

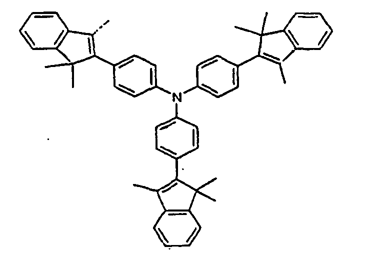

Zu einer Mischung aus 33.2 g (55 mmol) 4,4',4"-Tris(1H-inden-2-yl)triphenylamin und 300 ml Dimethylformamid werden 4.8 g (200 mmol) Natriumhydrid portionsweise zugegeben. Anschließend wird die Suspension auf 80°C erhitzt, bei dieser Temperatur tropfenweise mit 18.7 ml (300 mmol) Methyliodid versetzt und anschließend weitere 50 h bei 80 °C gerührt. Danach wird die Mischung bei Raumtemperatur mit 100 ml Ammoniak-Lösung und 500 ml Ethylacetat versetzt und die wässrige Phase abgetrennt. Die organische Phase wird fünfmal mit 300 ml Wasser gewaschen und anschließend über Na₂SO₄ getrocknet. Nach dem Entfernen des Lösemittels und mehrmaliger Umkristallisation aus Toluol (1.5 ml / g) erhält man gelbe Nadeln, die nach HPLC eine Reinheit von 99.8 % aufweisen. Die Ausbeute beträgt 23.7 g (60.3 %). Die Sublimation erfolgte bei einem Druck von ca. 5 x 10⁻⁵ mbar und T = 300°C.
¹H-NMR (CDCl₃, 500 MHz): δ [ppm] =1.51 (s, 18H), 2.10 (s, 9H), 7.14-7.20 (m, 9H), 7.24-7.27 (m, 6H), 7.40 (d, 3H), 7.47 (d, 3H), 7.58 (m, 3H).

### Beispiel 3: Synthese von 4,4',4"-Tris(spiro(fluoren-9,1'-inden-2-yl) triphenylamin (Amin 3)

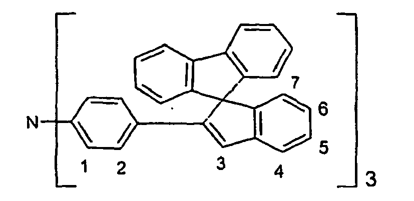

### a) Tris(4-(2-bromphenylvinyl)phenyl)amin

Eine auf 0 °C gekühlte Mischung von 107.5 g (350 mmol) 2-Brombenzyl-diethylphosphonat und 1000 ml DMF wird mit 67.3 g (700 mmol) Natrium-*tert*-butylat versetzt und 30 min. gerührt. Diese Mischung wird mit einer Lösung von 29.6 g (90 mmol) Tris(4-formyl-phenyl)amin in 1000 ml DMF bei 0°C während 30 min. versetzt und anschließend weitere 4 h bei 0°C gerührt. Dann wird unter Rühren mit 250 ml 2.5 N wässriger Salzsäure, anschließend mit 600 ml Wasser und dann mit 200 ml Ethanol versetzt und 30 min. nachgerührt. Der Niederschlag wird abgesaugt, zweimal mit je 200 ml eines Gemischs aus Wasser / Ethanol (1:1, v:v) und dreimal mit je 200 ml Ethanol gewaschen und anschließend im Vakuum getrocknet. Der so erhaltene Feststoff wird aus 160 ml DMF umkristallisiert, abschließend aus 500 ml Ethanol heiß ausgerührt und im Vakuum getrocknet.
Die Ausbeute bei einer Reinheit von 99.0 % n. ¹H-NMR beträgt 51.0 g (65 mmol) entsprechend 71.8% d. Th..
¹H-NMR (CDCl₃, 500 MHz): δ [ppm] = 7.66 (d, 3H), 7.58 (d, 3H), 7,47 (d, 6H), 7.39 (d, 3H), 7.30 (dd, 3H), 7.13 (d, 6H), 7.10 (dd, 3H), 7.01 (d, 3H).

### b) 4,4',4"-Tris(spiro(fluoren-9,1'-inden-2-yl)triphenylamin

Eine gut gerührte Suspension von 7.9 g (10 mmol) Tris(4-(2-bromphenylvinyl)-phenyl)amin in 300 ml Diethylether wird bei -78 °C tropfenweise mit 14.4 ml (36 mmol) n-Butyllithium (2.5 molar in n-Hexan) versetzt. Nach 15 min. Nachrühren wird das Kältebad entfernt, man lässt die Reaktionsmischung auf Raumtemperatur erwärmen und rührt anschließend weitere 3 h bei Raumtemperatur. Dann wird die Suspension bei Raumtemperatur mit einer Lösung von 7.2 g (40 mmol) Fluorenon in 200 ml Diethylether tropfenweise versetzt und weitere 14 h bei Raumtemperatur nachgerührt. Die gelbe Suspension wird mit einem Gemisch aus 10 ml Essigsäure und 200 ml Wasser versetzt und 30 min. gut durchgerührt. Die organische Phase wird abgetrennt, zweimal mit 500 ml Wasser gewaschen und zur Trockene eingeengt. Der Feststoff wird in 500 ml Toluol aufgenommen, mit 1.0 g p-Toluolsulfonsäure versetzt und so lange am Wasserabscheider gekocht, bis sich kein Wasser mehr abscheidet (ca. 3 h). Die gelbe Lösung wird nach Erkalten über Kieselgel filtriert, das Toluol wird im Vakuum entfernt und der Rückstand wird dreimal aus DMF (10 ml / g) und fünfmal aus Dioxan (10 ml / g) umkristallisiert.
Die Ausbeute bei einer Reinheit von 99.9 % n. ¹H-NMR beträgt 5.1 g (4.9 mmol) entsprechend 49.2 % d. Th.. Die Sublimation erfolgte bei einem Druck von ca. 5 x 10⁻⁵ mbar und T = 380 °C.
¹H-NMR (TCE-d2 + 1µl Hydrazinhydrat, 500 MHz): δ [ppm] = 7.81 (d, 6H, Fluoren), 7.42 (s, 3H, H3), 7.40 (d, 3H, H4), 7.32 (dd, 6H, Fluoren), 7.17 (dd, 3H, H5), 7.07 (dd, 6H, Fluoren), 6.87 (dd, 3H, H6), 6.81 (d, 6H, Fluoren), 6.76 (d, 6H, H2), 6.43 (d, 6H, H1), 6.38 (d, 3H, H7).

### c) Untersuchung zur Redox-Stabilität:

4,4',4"-Tris(spiro(fluoren-9,1'-inden-2-yl)triphenylamin wird in Lösung an Luft zum korrespondierenden Radikal-Kation oxidiert, erkennbar an einer starken Linienverbreiterung der ¹H-NMR-Signale des Phenyl-Indenyl-Teils des Spektrums. Die Reduktion zum Amin kann z. B. mit Hydrazinhydrat erfolgen (s. ¹H-NMR-Spektrum). Diese Redoxreaktion ist reversibel, es ist keine Zersetzung des Moleküls, auch bei wiederholter Durchführung des Redoxzyklus, zu beobachten.

### Beispiel 4: Synthese von 4,4',4"-Tris(1,1'-di-para-tolyl-inden-2-yl) triphenylamin (Amin 4)

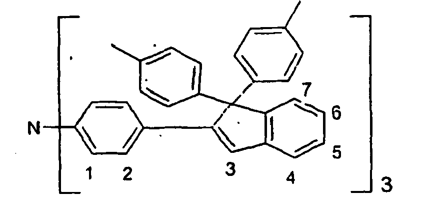

### a) 4,4',4"-Tris(1,1'-di-para-tolyl-inden-2-yl)triphenylamin

Eine gut gerührte Suspension von 7.9 g (10 mmol) Tris(4-(2-bromphenylvinyl)-phenyl)amin in 300 ml Dietylether wird bei -78 °C tropfenweise mit 14.4 ml (36 mmol) *n*-Butyllithium (2.5 molar in n-Hexan) versetzt. Nach 15 min. Nachrühren wird das Kältebad entfernt, man lässt die Reaktionsmischung auf Raumtemperatur erwärmen und rührt anschließend weitere 3 h bei Raumtemperatur. Dann wird die Suspension bei Raumtemperatur mit einer Lösung von 8.4 g (40 mmol) 4,4'-Dimethylbenzo-phenon in 200 ml Diethylether tropfenweise versetzt und weitere 14 h bei Raumtemperatur nachgerührt. Die gelbe Suspension wird mit einem Gemisch aus 10 ml Essigsäure und 200 ml Wasser versetzt und 30 min. gut durchgerührt. Die organische Phase wird abgetrennt, zweimal mit 500 ml Wasser gewaschen und zur Trockene eingeengt. Der Feststoff wird in 500 ml Toluol aufgenommen, mit 1.0 g p-Toluolsulfonsäure versetzt und so lange am Wasserabscheider gekocht, bis sich kein Wasser mehr abscheidet (ca. 3 h). Die gelbe Lösung wird nach Erkalten über Kieselgel filtriert, das Toluol wird im Vakuum entfernt und der Rückstand wird dreimal aus DMF (10 ml / g) und fünfmal aus Dioxan (8 ml / g) umkristallisiert.
Die Ausbeute bei einer Reinheit von 99.9 % n. ¹H-NMR beträgt 5.9 g (5.2 mmol) entsprechend 52.4 % d. Th. Die Sublimation erfolgte bei einem Druck von ca. 5 x 10⁻⁵ mbar und T = 370 °C.
¹H-NMR (TCE-d2 + 1µl Hydrazinhydrat, 500 MHz): δ [ppm] =7.40 (s, 3H, H3), 7.38 (d, 3H, H4), 7.20 (m, 12H, AB-p-Tolyl), 7.19 (dd, 3H, H5), 6.89 (dd, 3H, H6), 6.76 (d, 6H, H2), 6.62 (m, 12H, AB-p-Tolyl), 6.37 (d, 6H, H1), 6.36 (d, 3H, H7), 2.41 (s, 18H, CH₃).

### b) Untersuchung zur Redox-Stabilität:

4,4',4"-Tris(1,1'-di-*para*-tolyl-inden-2-yl) wird in Lösung an Luft zum korrespondierenden Radikal-Kation oxidiert, erkennbar an einer starken Linienverbreiterung der ¹H-NMR-Signale des Phenyl-Indenyl-Teils des Spektrums. Die Reduktion zum Amin kann z. B. mit Hydrazinhydrat erfolgen (s. ¹H-NMR-Spektrum). Diese Redoxreaktion ist reversibel, es ist keine Zersetzung des Moleküls, auch bei wiederholter Durchführung des Redoxzyklus, zu beobachten.

### Beispiel 5: Synthese von 4,4',4"-Tris[5,5,10,10-di-spiro-fluorenyl-5,10-dihydroindeno[2,1-alinden-2-yl]amin(Amin 5)

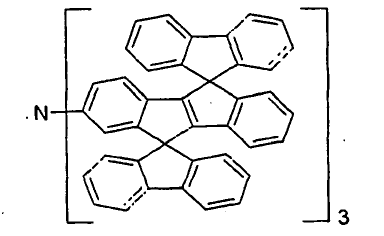

### a) 4,4',4"-Tris[2,3-dibrom-spiro(fluoren-9,1'-inden-2-yl)]triphenylamin

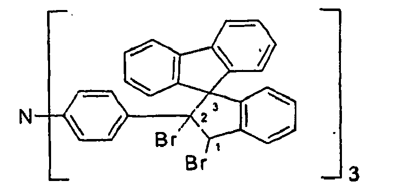

Eine auf 0 °C gekühlte Mischung von 51.9 g (50 mmol) 4,4',4"-Tris(spiro(fluoren-9,1'-inden-2-yl)triphenylamin (synthetisiert gemäß Beispiel 3) gelöst in 1000 ml Dichlormethan wird unter Lichtausschluss bei 0 °C mit einem Gemisch aus 2.6 ml (50 mmol) Brom und 100 ml Dichlormethan versetzt und 4 h bei 0 °C gerührt. Nach Entfernen des Dichlormethans im Vakuum wird der Rückstand mit etwas Ethanol gewaschen, einmal aus Toluol umkristallisiert und im Vakuum getrocknet. Die Ausbeute - bei einer Reinheit von etwa 97.0 % nach HPLC - beträgt 69.4 g (46 mmol), entsprechend 91.5 % d. Th.

### b) 4,4',4"-Tris[3-brom-spiro(fluoren-1',9-1 H-inden-2-yl)]triphenylamin

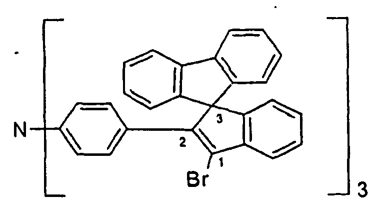

Eine gut gerührte Suspension von 40.0 g (26 mmol) 4,4',4"-Tris[2,3-dibrom-spiro(fluoren-9,1'-inden-2-yl)]triphenylamin in 300 ml THF wird bei 0 °C portionsweise mit 5.1 g (45 mmol) Kalium-*tert*-butanolat versetzt und 1 h bei Raumtemperatur gerührt. Anschließend gießt man die Mischung in 100 ml Eiswasser, extrahiert dreimal mit je 200 ml Dichlormethan, wäscht die Dichlormethan-Phase dreimal mit 300 ml Wasser, einmal mit 300 ml gesättigter NaCl-Lösung und trocknet dann über Natriumsulfat. Nach Entfernen des Lösungsmittel in Vakuum wird der Rückstand aus DMF umkristallisiert. Die Ausbeute - bei einer Reinheit von etwa 98.0 % nach HPLC - beträgt 28.7 g (22.5 mmol) entsprechend 85.4 % d. Th.

### c) 4,4',4"-Tris[5,5,10,10-di-spiro-fluorenyl-5,10-dihydroindeno[2,1-a]inden-2-yl]amin

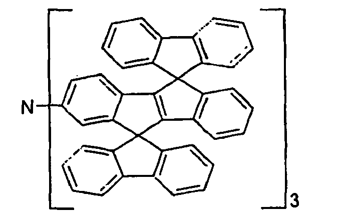

Eine gut gerührte-Suspension von 12.8 g (10 mmol) 4,4',4"-Tris[3-brom-spiro(fluoren-1',9-1H-inden-2-yl)]triphenylamin in 300 ml Diethylether wird bei -78 °C tropfenweise mit 14.4 ml (36 mmol) n-Butyllithium (2.5 molar in *n*-Hexan) versetzt. Nach 15 min. Nachrühren wird das Kältebad entfernt, man lässt die Reaktionsmischung auf Raumtemperatur erwärmen und rührt anschließend weitere 3 h bei Raumtemperatur. Dann wird die Suspension bei Raumtemperatur mit einer Lösung von 7.9 g (44 mmol) Fluorenon in 200 ml Diethylether tropfenweise versetzt und weitere 14 h bei Raumtemperatur nachgerührt. Die gelbe Suspension wird mit einem Gemisch aus 10 ml Essigsäure und 200 ml Wasser versetzt und 30 min. gut durchgerührt. Die organische Phase wird abgetrennt, zweimal mit 500 ml Wasser gewaschen und zur Trockene eingeengt. Der Feststoff wird in 500 ml Toluol aufgenommen, mit 500 mg p-Toluolsulfonsäure versetzt und so lange am Wasserabscheider gekocht, bis sich kein Wasser mehr abscheidet (ca. 3 h). Die gelbe Lösung wird nach Erkalten über Kieselgel filtriert, das Toluol wird im Vakuum entfernt und der Rückstand wird dreimal aus DMF (15 ml / g) und fünfmal Dioxan (12 ml / g) umkristallisiert. Die Ausbeute bei einer Reinheit von 99.9 % n. ¹H-NMR beträgt 7.5 g (4.9 mmol) entsprechend 49.2 % d. Th. Die Sublimation erfolgte bei einem Druck von ca. 5 x 10⁻⁵ mbar und T = 395 °C.
¹H-NMR (TCE-d2 + 1µl Hydrazinhydrat, 500 MHz): δ [ppm] = 7.81 (d, 6H, Fluoren), 7.77 (d, 6H, Fluoren), 7.40 (d, 3H), 7.32 (dd, 6H, Fluoren), 7.30 (dd, 6H, Fluoren), 7.17 (dd, 3H), 7.09 (d, 3H), 7.07 (dd, 6H, Fluoren), 7.01 (dd, 6H, Fluoren), 6.87 (dd, 3H), 6.85 (d, 6H, Fluoren), 6.80 (d, 6H, Fluoren), 6.66 (dd, 3H), 6.46 (dd, 3H), 6.38 (d, 3H), 6.28 (d, 3H).

### c) Untersuchung zur Redox-Stabilität:

4,4',4"-Tris[5,5,10,10-di-spiro-fluorenyl-5,10-dihydroindeno[2,1-a]inden-2-yl]amin wird in Lösung an Luft zum korrespondierenden Radikal-Kation oxidiert, erkennbar an einer starken Linienverbreiterung der ¹H-NMR-Signale des Phenyl-indenyl-Teils des Spektrums. Die Reduktion zum Amin kann z. B. mit Hydrazinhydrat erfolgen (s. ¹H-NMR-Spektrum). Diese Redoxreaktion ist reversibel, es ist keine Zersetzung des Moleküls, auch bei wiederholter Durchführung des Redoxzyklus, zu beobachten.

### Beispiel 6: Herstellung der OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 7 bis 11 werden die Ergebnisse verschiedener OLEDs vorgestellt. Der grundlegende Aufbau, die verwendeten Materialien und Schichtdicken, außer der emittierenden Schicht und der Elektronentransportschicht, sind in den Beispielen 7 bis 10 zur besseren Vergleichbarkeit identisch. Analog dem o. g. allgemeinen Verfahren werden OLEDs mit folgendem Aufbau erzeugt (wobei die unten aufgeführte Lochtransportschicht nicht für Beispiel 7 zutrifft):

| | |
|---|---|
| Lochinjektionsschicht (HIL) | 20 nm PEDOT (aus Wasser aufgeschleudert; bezogen von H. C. Starck, Goslar, Deutschland; Poly(3,4-ethylendioxy-2,5-thiophen)) |
| Lochtransportschicht (HTM) | 20 nm NaphDATA (aufgedampft; bezogen von SynTec, Wolfen, Deutschland; 4,4',4"-Tris(N-1-naphthyl-N-phenyl-amino)-triphenylamin) |
| Lochtransportschicht (HTM) | 20 nm S-TAD (aufgedampft; hergestellt nach WO 99/12888; 2,2',7,7'-Tetrakis(diphenylamino)-spiro-9,9'-bifluoren) |
| Emissionschicht (EML) | siehe Tabelle 1 für Materialien, Konzentration und Schichtdicken |
| Elektronenleiter (ETL) | siehe Tabelle 1 für Materialien und Schichtdicken |
| Ba-Al (Kathode) | 3 nm Ba, darauf 150 nm Al |

In Beispiel 7 werden als Lochtransportmaterialien, außer den beiden oben genannten, NPB (N-Naphthyl-N-phenyl-4,4'-diaminobiphenyl) und **HTM1** (2,2',7,7'-Tetrakis(di-paratolylamino)spiro-9,9'-bifluoren) verwendet.

Diese noch nicht optimierten OLEDs werden standardmäßig charakterisiert; hierfür werden die Elektrolumineszenzspektren, die Effizienz (gemessen in cd/A), die Leistungseffizienz (gemessen in Im/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit der OLED bei einer konstanten Stromdichte von 10 mA/cm² auf die Hälfte gesunken ist.

In Tabelle 1 sind die Ergebnisse einiger OLEDs (Beispiele 7 bis 10) zusammengefasst, wobei jeweils die Zusammensetzung der EML und der ETL inklusive der Schichtdicken mit aufgeführt ist. Die emittierenden Schichten enthalten als emittierende Materialien gemäß Formel (3) den Dotanden D1 (gemäß Strukturbeispiel 1). Als Vergleichsbeispiele dienen OLEDs, die als emittierende Verbindungen den Dotanden D2 gemäß dem oben genannten Stand der Technik bzw. nur das Hostmaterial enthalten. Als Hostmaterialien dienen die im Folgenden abgebildeten Verbindungen H1 bis **H4**. Als Elektronentransportmaterialien dienen **ETM1** (AlQ₃, bezogen von SynTec, Tris(chinolinato)aluminium(III)) oder **ETM2** (Bis(9,9'-spirobifluoren-2-yl)phenyl-phosphinoxid gemäß WO 05/003253).
In Tabelle 2 sind die Ergebnisse weiterer OLEDs (Beispiel 11) zusammengefasst, die als emittierende Materialien gemäß Formel (3) den Dotanden D1 bzw. den Dotanden **D3** enthalten und die durch Variation der Lochtransportschichten auf bessere Effizienz und Lebensdauer optimiert wurden.
Zur besseren Übersichtlichkeit sind die entsprechenden Strukturformeln der verwendeten Dotanden und Hostmaterialien im Folgenden dargestellt:

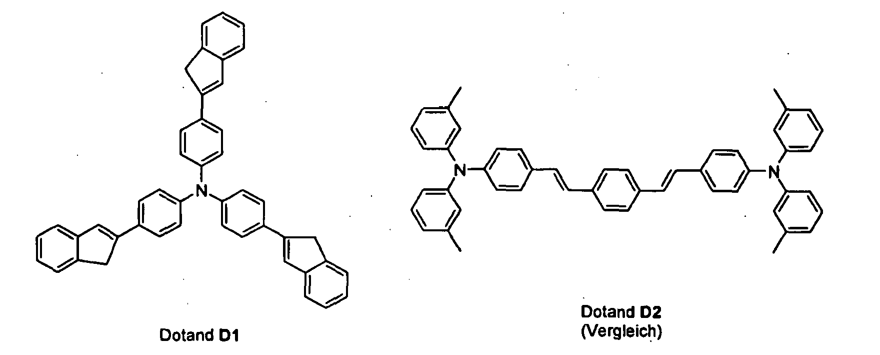

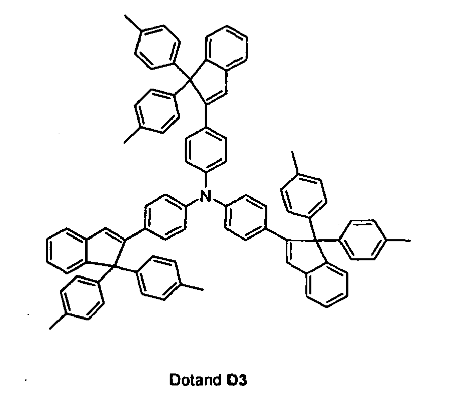

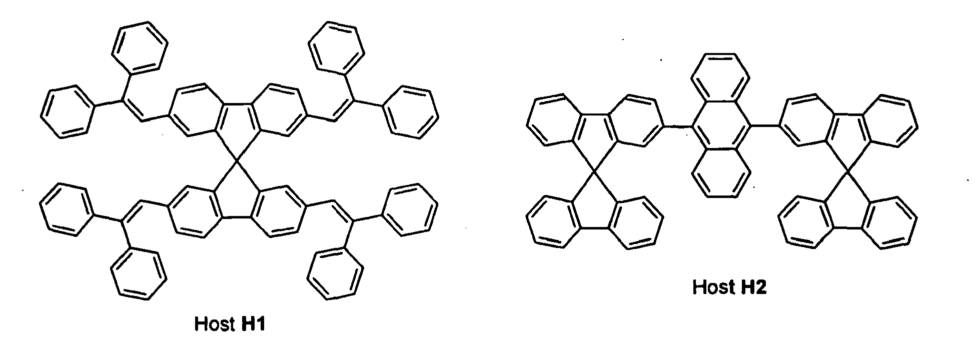

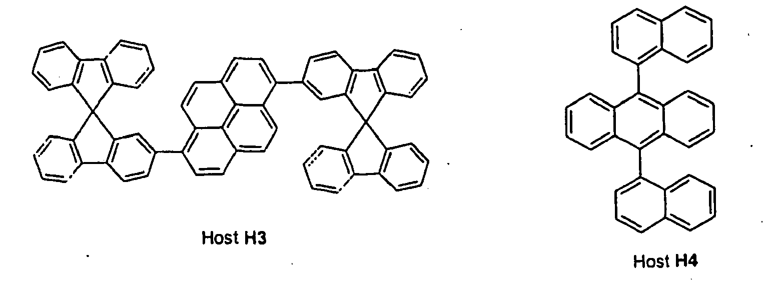

**Tabelle 1**

| Beispiel | EML | ETL | Max Effizienz | Spannung | CIE | Lebensdauer |
|---|---|---|---|---|---|---|
| | | | (cd/A) | (V) bei 100cd/m² | | (h) |
| **Beispiel 7a** | **H1** | **ETM1** | 4.2 | 5.8 | x=0.17; y=0.26 | 1200 |
| (Vergleich) | (30 nm) | (20 nm) | | | | |
| **Beispiel 7b** | **H1** : **D2** (5 %) | **ETM1** | 4.9 | 6.3 | x=0.17; y=0.31 | 1000 |
| (Vergleich) | (30 nm) | (20 nm) | | | | |
| **Beispiel 7c** | **H1: D1** (2 %) | **ETM1** | 4,0 | 4,8 | x=0.18; y=0.26 | 3100 |
| | (30 nm) | (20 nm) | | | | |
| **Beispiel 7d** | **H1 : D1** (5 %) | **ETM1** | 4.2 | 4.7 | x=0.19; y=0.28 | 2500 |
| | (30 nm) | (20 nm) | | | | |
| **Beispiel 7e** | **H1 : D1** (5 %) | **ETM2** | 4.0 | 4.6 | x=0.18; y=0.26 | 3600 |
| | (30 nm) | (20 nm) | | | | |
| **Beispiel 7f** | **H1 : D1** (5 %) | **ETM2** | 4,2 | 4.7 | x=0.16; y=0.25 | 3900 |
| | (30 nm) | (30 nm) | | | | |
| **Beispiel 8a** | **H2** | **ETM1** | 3.3 | 6.5 | x=0.15; y=0.15 | 700 |
| **(Vergleich)** | (30 nm) | (20 nm) | | | | |
| **Beispiel 8b** | **H2 : D1** (2 %) | **ETM1** | 3.9 | 5.2 | x=0.16; y=0.21 | 2500 |
| | (30 nm) | (20 nm) | | | | |
| **Beispiel 9a** | **H3** | **ETM1** | 3.7 | 5.8 | x=0.17; y=0.28 | 1400 |
| **(Vergleich)** | (30 nm) | (20 nm) | | | | |
| **Beispiel 9b** | **H3 : D2** (5 %) | **ETM1** | 5.4 | 6.5 | x=0.19; y=0.37 | 1000 |
| (Vergleich) | (30 nm) | (20 nm) | | | | |
| **Beispiel 9c** | **H3: D1** (2 %) | **ETM1** | 3.2 | 5.5 | x=0.17; y=0.18 | 2500 |
| | (30 nm) | (20 nm) | | | | |
| **Beispiel 9d** | **H3 : D1** (5 %) | **ETM1** | 3.5 | 5.3 | x=0.19; y=0.22 | 4100 |
| | (30 nm) | (20 nm) | | | | |
| **Beispiel 10a** | **H4** | **ETM1** | 1.1 | 5.8 | x=0:17; y=0.19 | 3100 |
| (Vergleich) | (30 nm) | (20 nm) | | | | |
| **Beispiel 10b** | **H4 : D1** (1 %) | **ETM1** | 3.8 | 5.1 | x=0.15; y=0.14 | 3000 |
| | (30 nm) | (20 nm) | | | | |
| **Beispiel 10c** | **H4 : D1** (2 %) | **ETM1** | 4.5 | 5.3 | x=0.15; y=0.16 | 3500 |
| | (30 nm) | (20 nm) | | | | |
| **Beispiel 10d** | **H4 : D1** (5 %) | **ETM1** | 4.0 | 4.8 | x=0.16; y=0.18 | 4000 |
| | (30 nm) | (20 nm) | | | | |
| **Beispiel 10e** | **H4 : D1** (2 %) | **ETM2** | 4,7 | 5.2 | x=0.15; y=0.15 | 5300 |
| | (30 nm) | (20 nm) | | | | |
| **Beispiel 10f** | **H4 : D1** (5 %) | **ETM2** | 4.3 | 4,6 | x=0.15; y=0.17 | 4800 |
| | (30 nm) | (20 nm) | | | | |

**Tabelle 2**

| **Beispiel** | HTL1 | HTL2 | EML | ETL | Max Effizienz | Spannung | CIE | Lebensdauer |
|---|---|---|---|---|---|---|---|---|
| | | | | | (cd/A) | (V) bei 100cd/m² | | (h) |
| **Beispiel 11a** | **HTM1** | **S-TAD** | **H4:** D1 (2%) | **ETM1** | 4.7 | 4.8 | x=0.15; y=0.15 | 5500 |
| | (20 nm) | (20 nm) | (30 nm) | (20 nm) | | | | |
| **Beispiel 11b** | **NaphDATA** | **NPB** | **H4** : D1 (2%) | **ETM1** | 4.9 | 5.0 | x=0.15; y=0.15 | 4500 |
| | (20 nm) | (20 nm) | (30 nm) | (20 nm) | | | | |
| **Beispiel 11c** | **HTM1** | **NPB** | **H4:** D1 (2%) | **ETM1** | 4.3 | 4.9 | x=0.15; y=0.15 | 6200 |
| | (20 nm) | (20 nm) | (30 nm) | (20 nm) | | | | |
| **Beispiel 12a** | **HTM1** | **S-TAD** | **H4** : D1 (2%) | **ETM1** | 3.8 | 4.9 | x=0.15; y=0.11 | 4000 |
| | (20 nm) | (20 nm) | (30 nm) | (20 nm) | | | | |
| **Beispiel 12b** | **NaphDATA** | **NPB** | **H4** : D1 (2%) | **ETM1** | 4.0 | 5.1 | x=0.15; y=0.11 | 3800 |
| | (20 nm) | (20 nm) | (30 nm) | (20 nm) | | | | |
| **Beispiel 12c** | **HTM1** | **NPB** | **H4** : D1 (2%) | **ETM1** | 3.5 | 5.2 | x=0.15; y=0.11 | 4300 |
| | (20 nm) | (20 nm) | (30 nm) | (20 nm) | | | | |

## Patentansprüche

1. Verbindungen gemäß Formel (3), 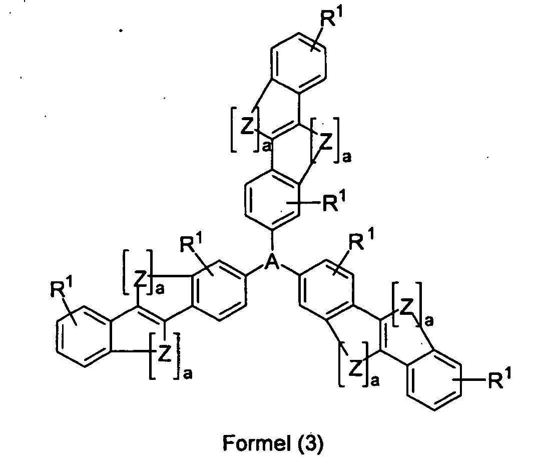 wobei für die verwendeten Symbole gilt:
A ist bei jedem Auftreten gleich oder verschieden N, P oder P=O;
Z ist bei jedem Auftreten gleich oder verschieden eine bivalente Gruppe C(R¹)2, C=O, C[=C(R¹)₂], Si(R¹)₂, O, S=O, SO₂, NR, BR¹, PR¹, PR¹O, C(R¹)₂-C(R¹)₂, C(R¹)2-NR¹, C(R¹)₂-C(R¹)₂-C(R¹)₂ oder C(R¹)₂-O-C(R¹)₂;
R¹ ist bei jedem Auftreten gleich oder verschieden H, F, Cl, Br, I, CN, NO₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch -R²C=CR²-, -C≡C-, P(=O)(R²), SO, SO₂, Si(R²)₂, Ge(R²)₂, Sn(R²)₂, C=O, C=S, C=Se, C=NR², -O-, -S- oder -CONR²- ersetzt sein können und wobei ein oder mehrere H-Atome durch F, Cl, Br, I, CN oder NO₂ ersetzt sein können, oder ein aromatisches oder heteroaromatisches System mit 5 bis 40 aromatischen Ringatomen, das durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Kombination aus zwei, drei oder vier dieser Systeme; dabei können zwei oder mehrere Substituenten R¹ auch miteinander ein mono- oder polycyclisches aliphatisches Ringsystem bilden;
R² ist bei jedem Auftreten gleich oder verschieden H oder ein aliphatischer oder aromatischer Kohlenwasserstoffrest mit 1 bis 20 C-Atomen;
a ist bei jedem Auftreten gleich oder verschieden 0 oder 1, mit der Maßgabe, dass pro Doppelbindung mindestens ein Index a = 1 ist, wobei a = 0 bedeutet, dass statt Z eine Gruppe R¹ gebunden ist;
wobei die maximal Anzahl der Substituenten R¹ der Anzahl der substituierbaren H-Atome entspricht.

2. Verbindungen gemäß Anspruch 1, **dadurch gekennzeichnet, dass** für die Symbole und Indizes gilt:
A ist bei jedem Auftreten N oder P;
Z ist bei jedem Auftreten gleich oder verschieden C(R¹)₂, SO₂, BR¹, P(R¹)O, C(R¹)₂-C(R¹)₂, C(R¹)₂-NR¹;
a ist bei jedem Auftreten gleich 0 oder 1, wobei an jeder Doppelbindung ein Index a = 0 und der andere Index a = 1 ist;
R¹ und R² sind wie unter Anspruch 1 definiert.

3. Verbindungen gemäß Anspruch 2, **dadurch gekennzeichnet, dass** für die Symbole und Indizes gilt:
A ist bei jedem Auftreten N;
Z ist bei jedem Auftreten gleich oder verschieden C(R¹)₂, P(R¹)O oder C(R¹)₂-C(R¹)₂;
R¹, R² und a sind wie unter Anspruch 2 definiert.

4. Verbindungen gemäß einem oder mehreren der Ansprüche 1 bis 3, **dadurch gekennzeichnet, dass** alle Einheiten Z jeweils gleich gewählt sind.

5. Verbindungen gemäß einem oder mehreren der Ansprüche 1 bis 4, **dadurch gekennzeichnet, dass** sie symmetrisch aufgebaut sind und eine dreizählige Drehachse aufweisen.

6. Verbindungen gemäß einem oder mehreren der Ansprüche 1 bis 5, **dadurch gekennzeichnet, dass** diese aus den Strukturen (1) bis (30) ausgewählt sind, die mit R¹ substituiert oder unsubstituiert sein können:

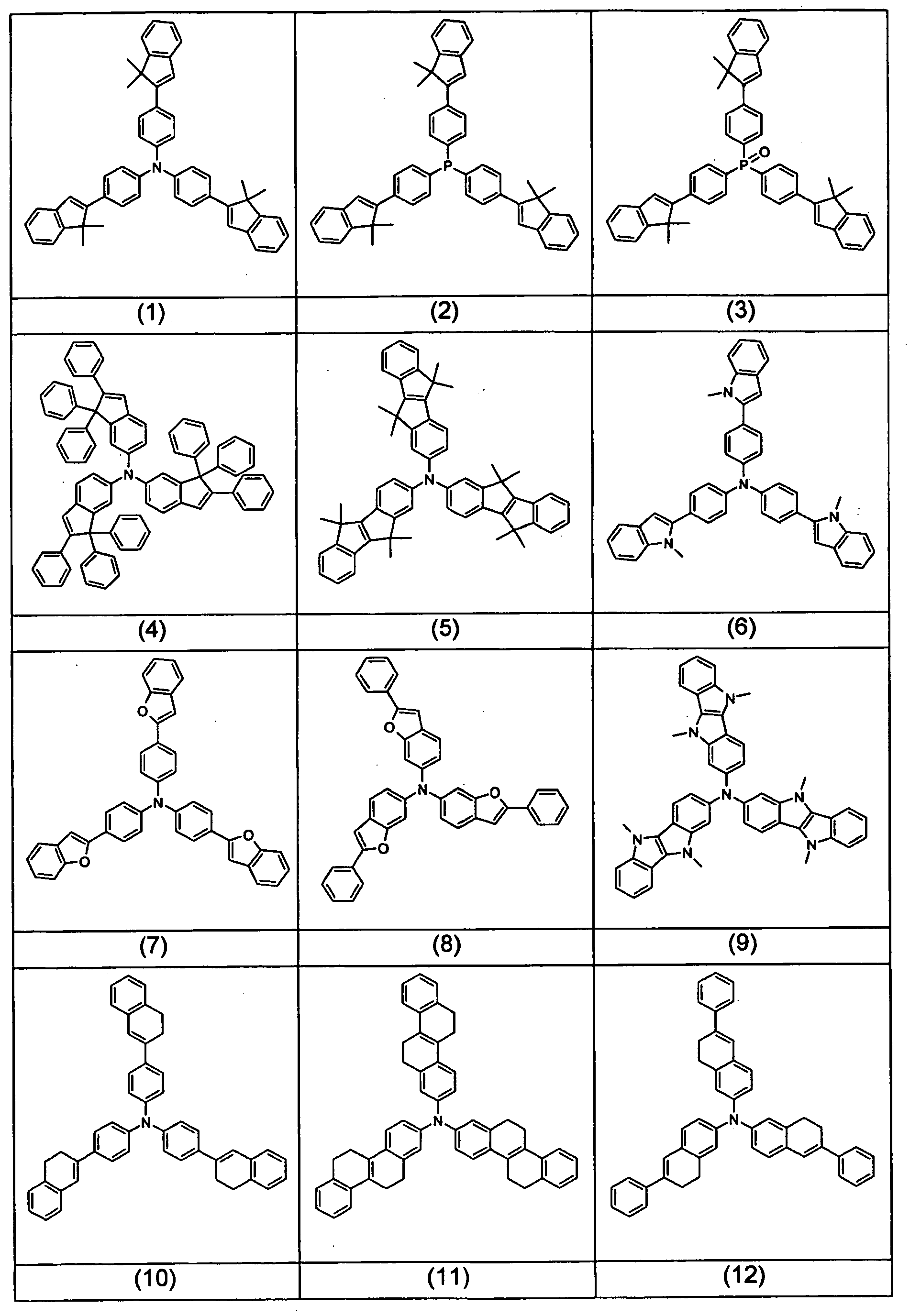

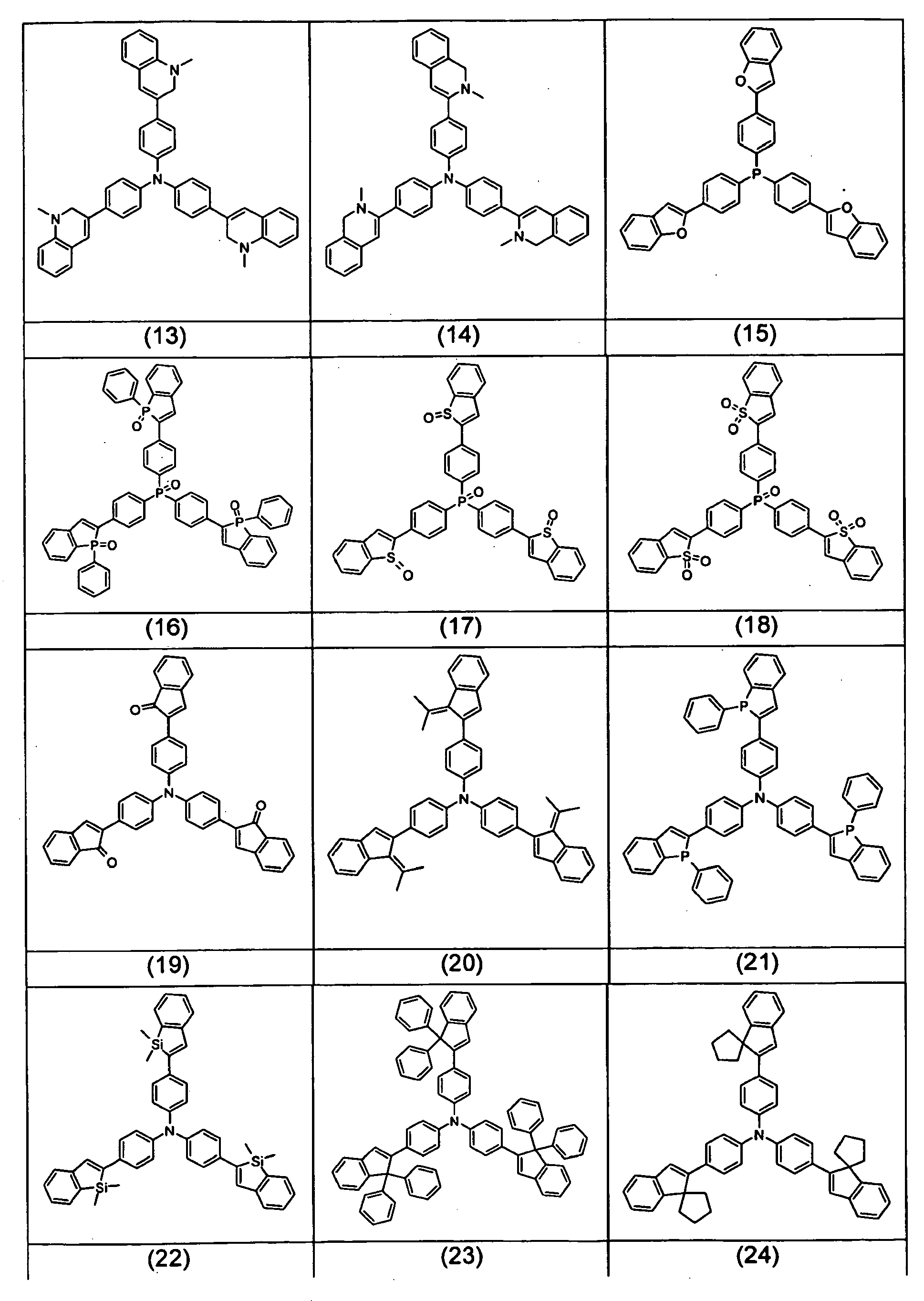

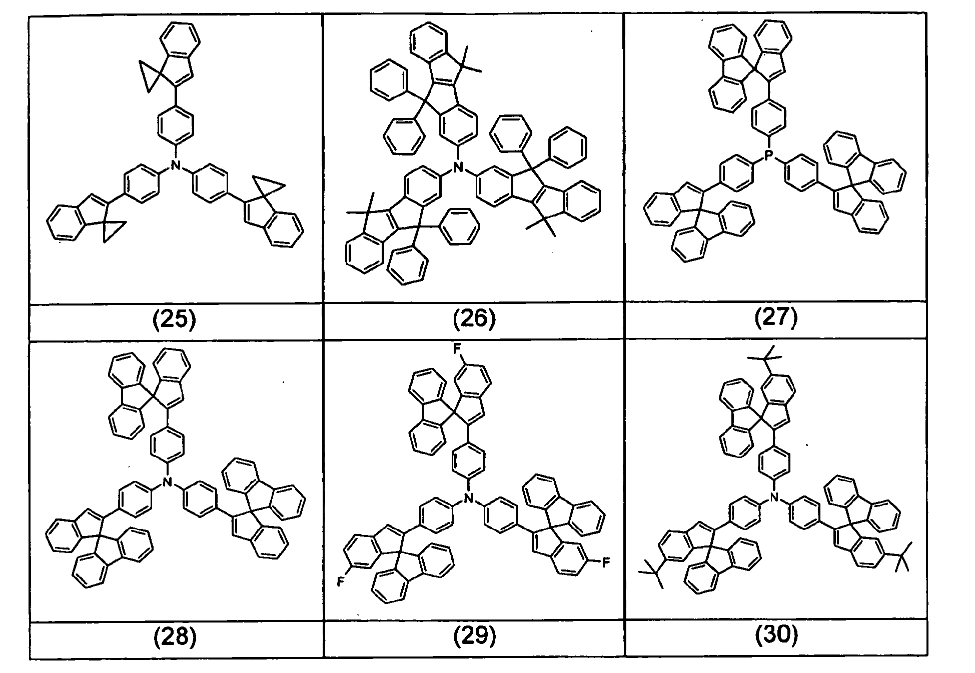

7. Verwendung von Verbindungen gemäß einem oder mehreren der Ansprüche 1 bis 6 in organischen elektronischen Vorrichtungen.

8. Organische Elektrolumineszenzvorrichtungen, enthaltend Kathode, Anode und mindestens eine emittierende Schicht, **dadurch gekennzeichnet, dass** mindestens eine organische Schicht mindestens eine Verbindung gemäß einem oder mehreren der Ansprüche 1 bis 6 enthält.

9. Organische Elektrolumineszenzvorrichtung gemäß Anspruch 8, **dadurch gekennzeichnet, dass** sie außer der emittierenden Schicht Lochinjektionsschicht, Lochtransportschicht, Lochblockierschicht, Elektronentransportschicht und/oder Elektroneninjektionsschicht enthält.

10. Organische Elektrolumineszenzvorrichtungen gemäß Anspruch 8 und/oder 9, **dadurch gekennzeichnet, dass** die Verbindung gemäß einem oder mehreren der Ansprüche 1 bis 6 als Emitter verwendet wird und für die Symbole A und Z gilt:
A ist bei jedem Auftreten Stickstoff;
Z ist bei jedem Auftreten gleich oder verschieden C(R¹)₂, C(R¹)₂-C(R¹)₂ oder C(R¹)₂-NR¹.

11. Organische Elektrolumineszenzvorrichtungen gemäß Anspruch 8 und/oder 9, **dadurch gekennzeichnet, dass** die Verbindung gemäß einem oder mehreren der Ansprüche 1 bis 6 als Lochtransportmaterial verwendet wird und für die Symbole A und Z gilt:
A ist bei jedem Auftreten Stickstoff oder Phosphor;
Z ist bei jedem Aufteten gleich oder verschieden C(R¹)₂, C(R¹)₂-C(R¹)₂, C(R¹)₂-NR¹, O, NR¹ oder PR¹.

12. Organische Elektrolumineszenzvorrichtungen gemäß Anspruch 8 und/oder 9, **dadurch gekennzeichnet, dass** die Verbindung gemäß einem oder mehreren der Ansprüche 1 bis 6 als Elektronentransportmaterial verwendet wird und für die Symbole A und Z gilt:
A ist bei jedem Auftreten P=O;
Z ist bei jedem Auftreten gleich oder verschieden C(R¹)₂, C(R¹)₂-C(R¹)₂, C(R¹)₂-NR¹, C=O, S=O, SO₂, BR¹ oder PR¹O.

13. Organische Elektrolumineszenzvorrichtungen gemäß Anspruch 8 und/oder 9, **dadurch gekennzeichnet, dass** die Verbindung gemäß einem oder mehreren der Ansprüche 1 bis 6 als Matrixmaterial oder als Lochblockiermaterial in elektrophosphoreszierenden Vorrichtungen verwendet wird und für die Symbole A und Z gilt:
A ist bei jedem Auftreten P=O;
Z ist bei jedem Auftreten gleich oder verschieden C(R¹)₂, C(R¹)₂-C(R¹)₂, C(R¹)₂-NR¹, C=O, S=O, SO₂ oder PR¹O.

14. Organische Feld-Effekt-Transistoren (O-FETs), organische Dünnfilmtransistoren (O-TFTs), organische integrierte Schaltungen (O-ICs), organische Solarzellen (O-SCs) und organische Laserdioden (O-Laser), enthaltend eine oder mehrere Verbindungen gemäß einem oder mehreren der Ansprüche 1 bis 6.

## Claims

1. Compounds of the formula (3) 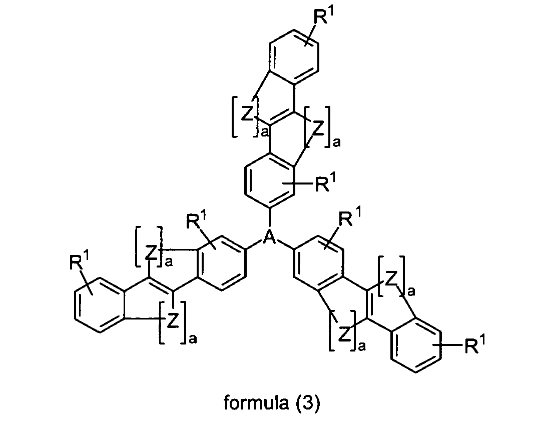 where the following applies to the symbols used:
A is on each occurrence, identically or differently, N, P or P=O;
Z is on each occurrence, identically or differently, a divalent group C(R¹)₂, C=O, C[=C(R¹)₂], Si(R¹)₂, O, S=O, SO₂, NR, BR¹, PR¹, PR¹O, C(R¹)₂- C(R¹)₂, C(R¹)₂-NR¹, C(R¹)₂-C(R¹)₂-C(R¹)₂ or C(R¹)₂-O-C(R¹)₂;
R¹ is on each occurrence, identically or differently, H, F, Cl, Br, I, CN, NO₂, a straight-chain alkyl, alkoxy or thioalkoxy group having 1 to 40 C atoms or a branched or cyclic alkyl, alkoxy or thioalkoxy group having 3 to 40 C atoms, each of which may be substituted by one or more radicals R², where one or more non-adjacent CH₂ groups may be replaced by -R²C=CR²-, -C≡C-, P(=O)(R²), SO, SO₂, Si(R²)₂, Ge(R²)₂, Sn(R²)₂, C=O, C=S, C=Se, C=NR², -O-, -S- or -CONR²- and where one or more H atoms may be replaced by F, Cl, Br, I, CN or NO₂, or an aromatic or heteroaromatic system having 5 to 40 aromatic ring atoms, which may be substituted by one or more radicals R², or an aryloxy or heteroaryloxy group having 5 to 40 aromatic ring atoms, which may be substituted by one or more radicals R², or a combination of two, three or four of these systems; two or more substituents R¹ here may also form a mono- or polycyclic aliphatic ring system with one another;
R² is on each occurrence, identically or differently, H or an aliphatic or aromatic hydrocarbon radical having 1 to 20 C atoms;
a is on each occurrence, identically or differently, 0 or 1, with the proviso that at least one index a = 1 per double bond, where a = 0 means that a group R¹ is bonded instead of Z;
where the maximum number of substituents R¹ corresponds to the number of substitutable H atoms.

2. Compounds according to Claim 1, **characterised in that** the following applies to the symbols and indices:
A is on each occurrence N or P;
Z is on each occurrence, identically or differently, C(R¹)₂, SO₂, BR¹, P(R¹)O, C(R¹)₂-C(R¹)₂, C(R¹)₂-NR¹;
a is on each occurrence equal to 0 or 1, where for each double bond, one index a is equal to zero and the other is equal to one;
R¹ and R² are as defined under Claim 1.

3. Compounds according to Claim 2, **characterised in that** the following applies to the symbols and indices:
A is on each occurrence N;
Z is on each occurrence, identically or differently, C(R¹)₂, P(R¹)O or C(R¹)₂-C(R¹)₂;
R¹, R² and a are as defined under Claim 2.

4. Compounds according to one or more of Claims 1 to 3, **characterised in that** all units Z are in each case selected identically.

5. Compounds according to one or more of Claims 1 to 4, **characterised in that** they have a symmetrical structure and a three-fold axis of rotation.

6. Compounds according to one or more of Claims 1 to 5, **characterised in that** they are selected from structures (1) to (30) which may be substituted by R¹ or unsubstituted:

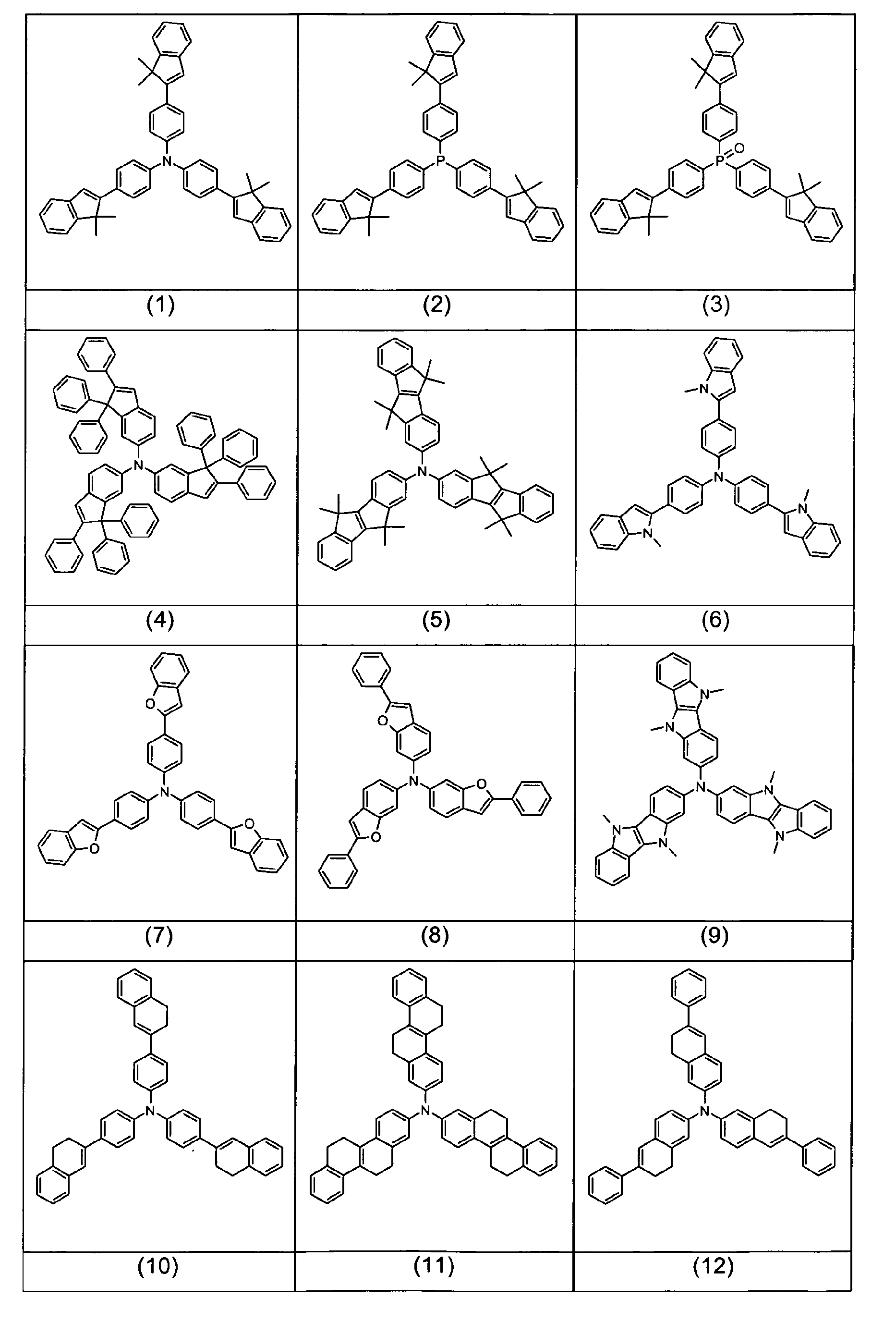

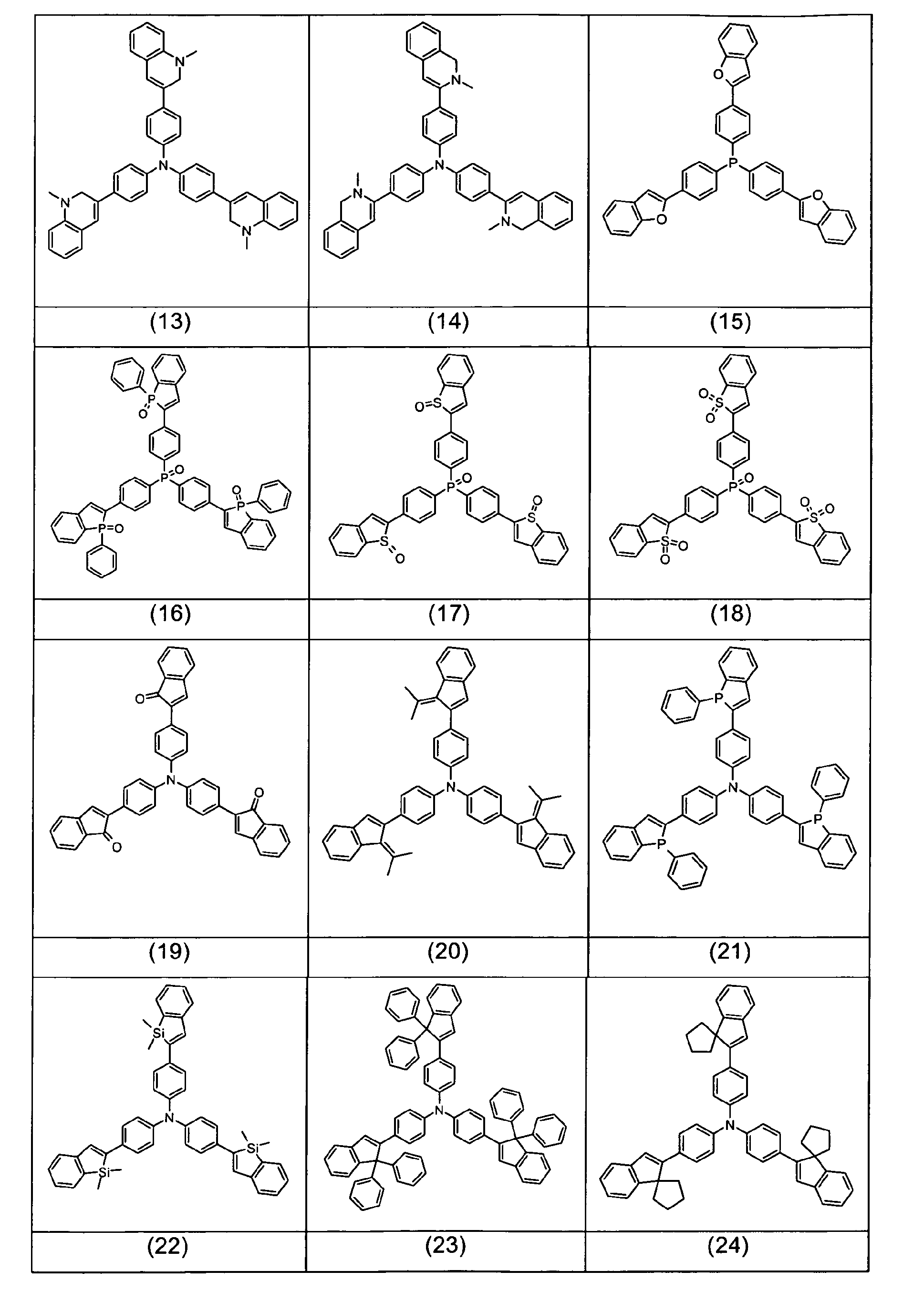

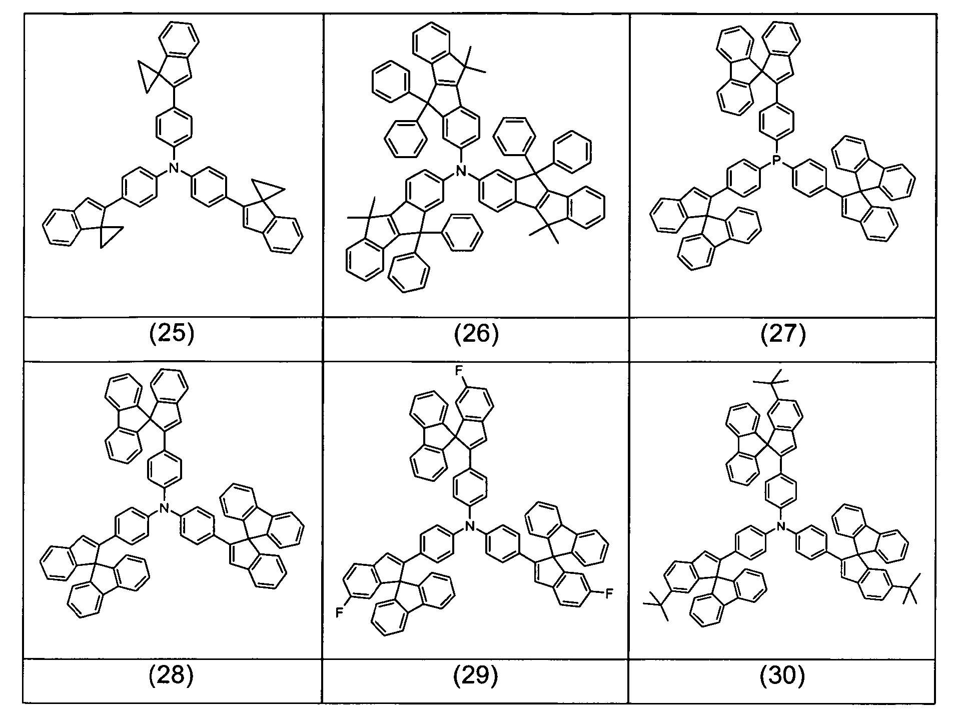

7. Use of compounds according to one or more of Claims 1 to 6 in organic electronic devices.

8. Organic electroluminescent devices comprising cathode, anode and at least one emitting layer, **characterised in that** at least one organic layer comprises at least one compound according to one or more of Claims 1 to 6.

9. Organic electroluminescent devices according to Claim 8, **characterised in that**, in addition to the emitting layer, it comprises a hole-injection layer, hole-transport layer, hole-blocking layer, electron-transport layer and/or electron-injection layer.

10. Organic electroluminescent devices according to Claim 8 and/or 9, **characterised in that** the compound according to one or more of Claims 1 to 6 is used as emitter and the following applies to the symbols A and Z:
A is on each occurrence nitrogen;
Z is on each occurrence, identically or differently, C(R¹)₂, C(R¹)₂-C(R¹)₂ or C(R¹)₂-NR¹.

11. Organic electroluminescent devices according to Claim 8 and/or 9, **characterised in that** the compound according to one or more of Claims 1 to 6 is used as hole-transport material and the following applies to the symbols A and Z:
A is on each occurrence nitrogen or phosphorus;
Z is on each occurrence, identically or differently, C(R¹)₂, C(R¹)₂-C(R¹)₂, C(R¹)₂-NR¹, O, NR¹ or PR¹.

12. Organic electroluminescent devices according to Claim 8 and/or 9, **characterised in that** the compound according to one or more of Claims 1 to 6 is used as electron-transport material and the following applies to the symbols A and Z:
A is on each occurrence P=O;
Z is on each occurrence, identically or differently, C(R¹)₂, C(R¹)₂-C(R¹)₂, C(R¹)₂-NR¹, C=O, S=O, SO₂, BR¹ or PR¹O.

13. Organic electroluminescent devices according to Claim 8 and/or 9, **characterised in that** the compound according to one or more of Claims 1 to 6 is used as matrix material or as hole-blocking material in electrophosphorescent devices and the following applies to the symbols A and Z:
A is on each occurrence P=O;
Z is on each occurrence, identically or differently, C(R¹)₂, C(R¹)₂-C(R¹)₂, C(R¹)₂-NR¹, C=O, S=O, SO₂ or PR¹O.

14. Organic field-effect transistors (O-FETs), organic thin-film transistors (O-TFTs), organic integrated circuits (O-ICs), organic solar cells (O-SCs) and organic laser diodes (O-lasers) comprising one or more compounds according to one or more of Claims 1 to 6.

## Revendications

1. Composés de la formule (3), 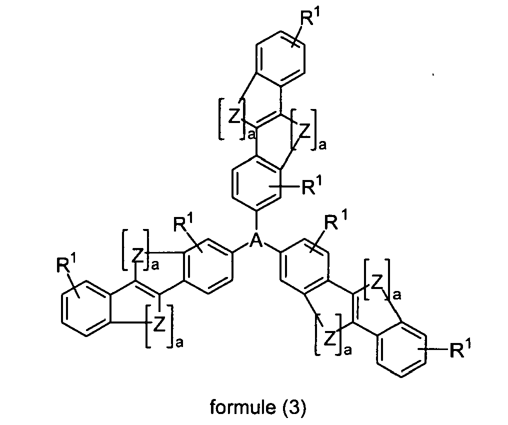 dans laquelle ce qui suit s'applique aux symboles utilisés :
A est à chaque occurrence, de façon identique ou différente, N, P ou P=O ;
Z est à chaque occurrence, de façon identique ou différente, un groupe di- valent C(R¹)₂, C=O, C[=C(R¹)₂], Si(R¹)₂, O, S=O, SO₂, NR, BR¹, PR¹, PR¹O, C(R¹)₂-C(R¹)₂, C(R¹)₂-NR¹, C(R¹)₂-C(R¹)₂-C(R¹)₂ ou C(R¹)₂-O- C(R¹)₂ ;
R¹ est à chaque occurrence, de façon identique ou différente, H, F, Cl, Br, I, CN, NO₂, un groupe alkyle, alcoxy ou thioalcoxy en chaîne droite ayant 1 à 40 atomes de C ou un groupe alkyle, alcoxy ou thioalcoxy ramifié ou cyclique ayant 3 à 40 atomes de C, dont chacun peut être substitué par un ou plusieurs radicaux R², où un ou plusieurs groupes CH₂ non adja- cents peuvent être remplacés par -R²C=CR²-, -C≡C-, P(=O)(R²), SO, SO₂, Si(R²)_{2,} Ge(R²)₂, Sn(R²)₂, C=O, C=S, C=Se, C=NR², -O-, -S- ou -CONR²- et où un ou plusieurs atomes de H peuvent être remplacés par F, Cl, Br, I, CN ou NO₂, ou un système aromatique ou hétéroaromatique ayant 5 à 40 atomes de cycle aromatique, qui peut être substitué par un ou plusieurs radicaux R², ou un groupe aryloxy ou hétéroaryloxy ayant 5 à 40 atomes de cycle aromatique, qui peut être substitué par un ou plu- sieurs radicaux R², ou une combinaison de deux, trois ou quatre de ces systèmes ; deux ou plus de deux substituants R¹ peuvent former ici également un système de cycle aliphatique mono- ou polycyclique avec un autre ;
R² est à chaque occurrence, de façon identique ou différente, H ou un radi- cal hydrocarbure aliphatique ou aromatique ayant 1 à 20 atomes de C ;
a est à chaque occurrence, de façon identique ou différente, 0 ou 1, sous réserve qu'au moins un indice a = 1 par double liaison, où a = 0 signifie qu'un groupe R¹ est lié à la place de Z ;
où le nombre maximum de substituants R¹ correspond au nombre d'atomes de H substituables.

2. Composés selon la revendication 1, **caractérisés en ce** qui sui s'applique aux symboles et indices :
A est à chaque occurrence N ou P ;
Z est à chaque occurrence, de façon identique ou différente, C(R¹)₂, SO₂, BR¹, P(R¹)O, C(R¹)₂-C(R¹)₂, C(R¹)₂-NR¹;
a est à chaque occurrence égal à 0 ou 1, où un indice a = 0 et l'autre indice a = 1 sur chaque double liaison ;
R¹ et R² sont comme définis dans la revendication 1.

3. Composés selon la revendication 2, **caractérisés en ce** qui sui s'applique aux symboles et indices :
A est à chaque occurrence N ;
Z est à chaque occurrence, de façon identique ou différente, C(R¹)₂, P(R¹)O ou C(R¹)₂-C(R¹)₂;
R¹, R² et a sont comme définis dans la revendication 2.

4. Composés selon une ou plusieurs des revendications 1 à 3, **caractérisés en ce que** toutes les unités Z sont dans chaque cas choisies de façon identique.

5. Composés selon une ou plusieurs des revendications 1 à 4, **caractérisés en ce qu'**ils ont une structure symétrique et un axe de rotation replié trois fois.

6. Composés selon une ou plusieurs des revendications 1 à 5, **caractérisés en ce qu'**ils sont choisis parmi les structures (1) à (30) qui peuvent être substituées par R¹ ou non substituées :

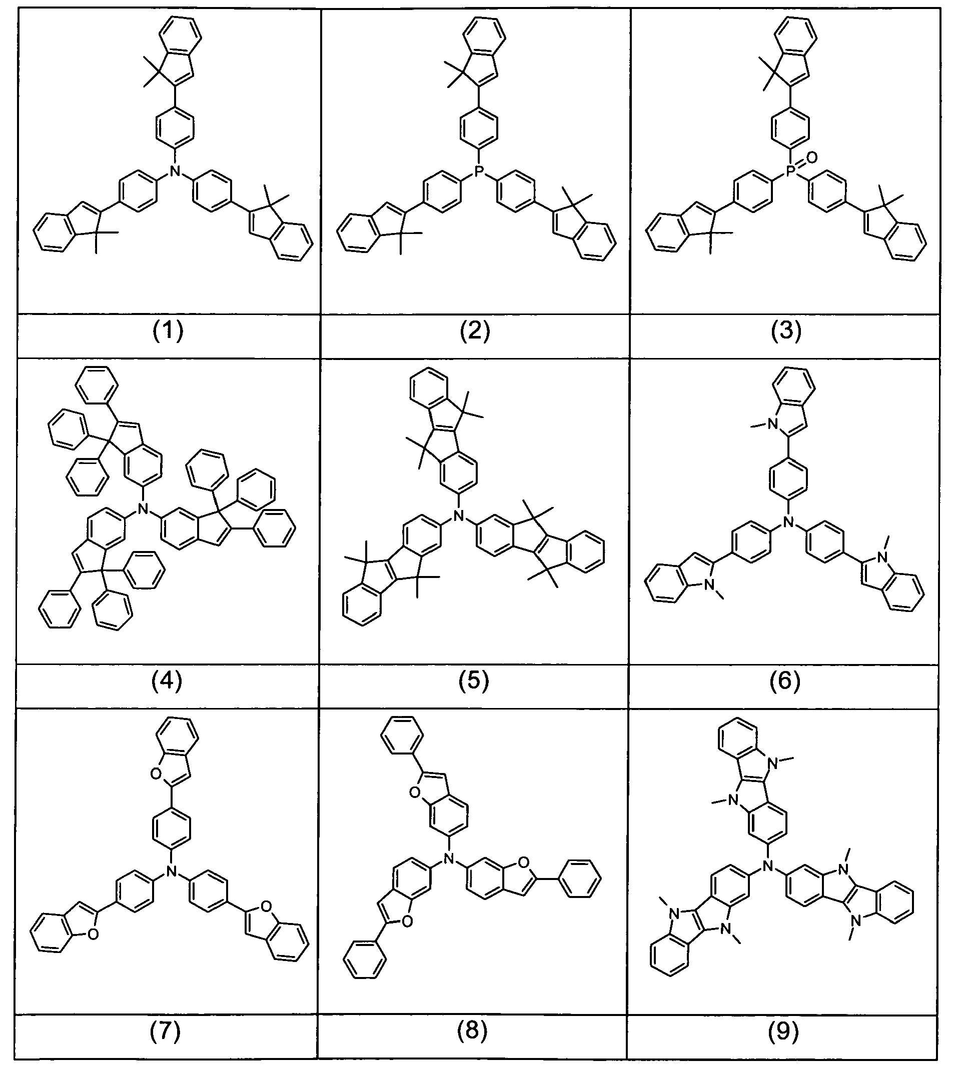

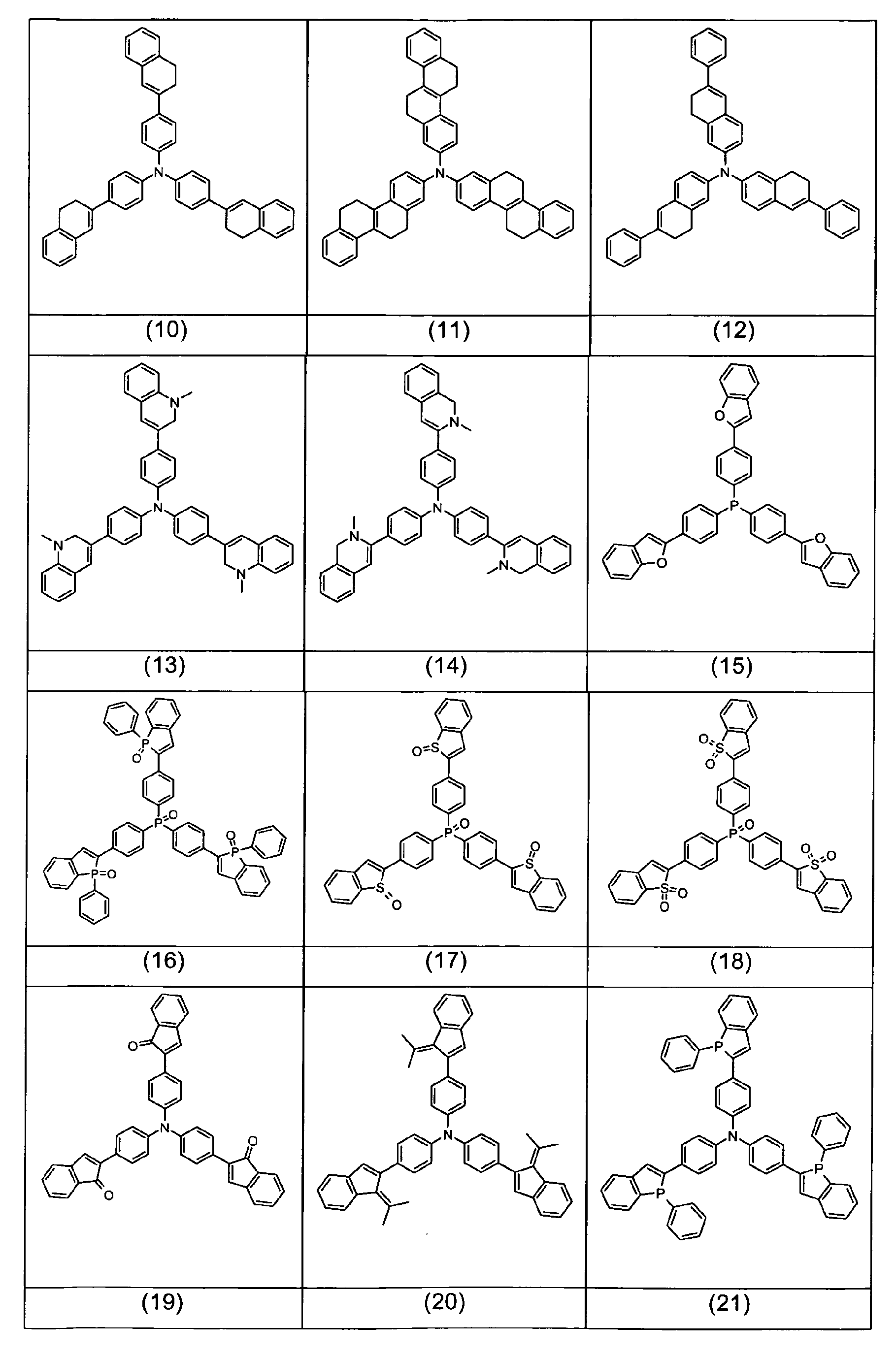

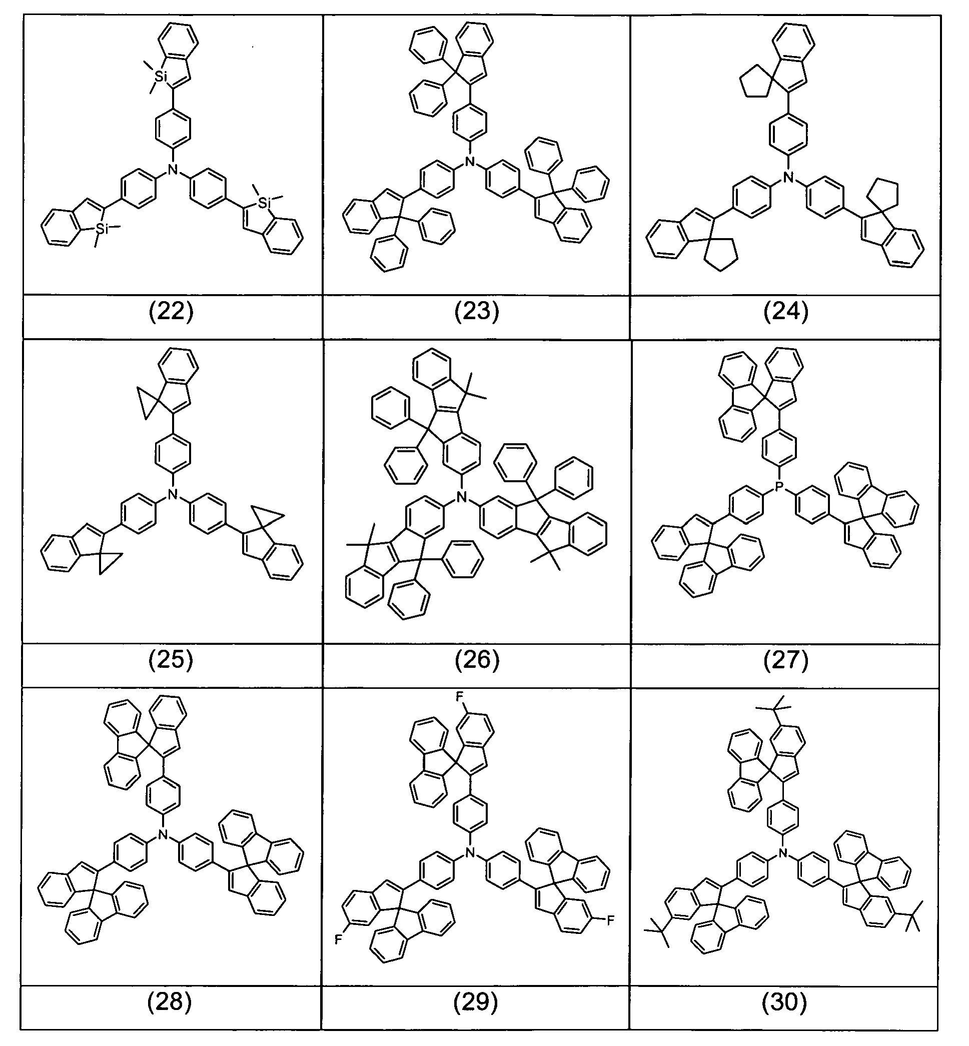

7. Utilisation de composés selon une ou plusieurs des revendications 1 à 6 dans des dispositifs électroniques organiques.

8. Dispositifs électroluminescents organiques comprenant une cathode, une anode et au moins une couche émettrice, **caractérisés en ce qu'**au moins une couche organique comprend au moins un composé selon une ou plusieurs des revendications 1 à 6.

9. Dispositifs électroluminescents organiques selon la revendication 8, **caractérisés en ce que**, indépendamment de la couche émettrice, ils comprennent une couche d'injection de trous, une couche de transport de trous, une couche de blocage de trous, une couche de transport d'électrons et/ou une couche d'injection d'électrons.

10. Dispositifs électroluminescents organiques selon la revendication 8 et/ou 9, **caractérisés en ce que** le composé selon une ou plusieurs des revendications 1 à 6 est utilisé comme émetteur et ce qui suit s'applique aux symboles A et Z :
A est à chaque occurrence azote ;
Z est à chaque occurrence, de façon identique ou différente, C(R¹)₂, C(R¹)₂- C(R¹)₂ ou C(R¹)₂-NR¹.

11. Dispositifs électroluminescents organiques selon la revendication 8 et/ou 9, **caractérisés en ce que** le composé selon une ou plusieurs des revendications 1 à 6 est utilisé comme matériau de transport de trous et ce qui suit s'applique aux symboles A et Z :
A est à chaque occurrence azote ou phosphore ;
Z est à chaque occurrence, de façon identique ou différente, C(R¹)₂, C(R¹)₂- C(R¹)₂, C(R¹)₂-NR¹, O, NR¹ ou PR¹.

12. Dispositifs électroluminescents organiques selon la revendication 8 et/ou 9, **caractérisés en ce que** le composé selon une ou plusieurs des revendications 1 à 6 est utilisé comme matériau de transport d'électrons et ce qui suit s'applique aux symboles A et Z :
A est à chaque occurrence P=O ;
Z est à chaque occurrence, de façon identique ou différente, C(R¹)₂, C(R¹)₂-C(R¹)₂, C(R¹)₂-NR¹, C=O, S=O, SO₂, BR¹ ou PR¹O.

13. Dispositifs électroluminescents organiques selon la revendication 8 et/ou 9, **caractérisés en ce que** le composé selon une ou plusieurs des revendications 1 à 6 est utilisé comme matériau de matrice ou comme matériau de blocage de trous dans des dispositifs électrophosphorescents et ce qui suit s'applique aux symboles A et Z :
A est à chaque occurrence P=O ;
Z est à chaque occurrence, de façon identique ou différente, C(R¹)₂, C(R¹)₂-C(R¹)₂, C(R¹)₂-NR¹, C=O, S=O, SO₂ ou PR¹O.

14. Transistors à effet de champ organiques (O-FETs), transistors à film mince organiques (O-TFTs), circuits intégrés organiques (O-ICs), cellules solaires organiques (O-SCs) et diodes laser organiques (O-lasers) comprenant un ou plusieurs composés selon une ou plusieurs des revendications 1 à 6.
